# EUROPEAN PATENT APPLICATION

(11) **EP 4 249 583 A1**
(43) Date of publication of application: **27.09.2023**
(21) Application number: 21893988.2
(22) Date of filing: 18.11.2021
(51) Int. Cl.: C12N 1/19, C12P 7/64, C12R 1/85

(54) **USE OF FATTY ACID ELONGASE GENE AND ESTERASE GENE IN SYNTHESIS OF NERVONIC ACID AND GREASE IN YEAST**

(30) Priority: 19.11.2020 CN 202011302605
(71) Applicant: Qingdao Institute of Bioenergy and Bioprocess Technology, Chinese Academy of Sciences, Qingdao, Shandong 266101 (CN); Zhejiang Zhenyuan Biotech Co., Ltd., Shaoxing, Zhejiang 312365 (CN)
(72) Inventor: WANG, Shian, Qingdao, Shandong 266101 (CN); RUAN, Jianchang, Shaoxing, Zhejiang 312071 (CN); LI, Fuli, Qingdao, Shandong 266101 (CN); SU, Hang, Qingdao, Shandong 266101 (CN); FAN, Weiming, Shaoxing, Zhejiang 312071 (CN); HAN, Xingfeng, Qingdao, Shandong 266101 (CN); MENG, Ziyue, Qingdao, Shandong 266101 (CN); SHI, Penghui, Qingdao, Shandong 266101 (CN)
(74) Representative: Santoro, Sofia
(86) International application number: PCT/CN2021/131563
(87) International publication number: WO 2022/105841

(57) **Abstract**

Provided is an engineered bacterium for producing nervonic acid and/or grease. The genome of the engineering bacterium is integrated with an expression cassette expressing a protein encoded by 3-ketoacyl-CoA synthase (KCS) gene and/or an exterase gene.

## Description

### Technical solution

The present invention relates to the field of biotechnology, and more specifically to the application of a fatty acid elongase gene and an esterifying enzyme gene in the synthesis of nervonic acid in yeast.

### Background

Nervonic acid (cis-15-Tetracosenoic acid, 24:1Δ15) is a very long chain monounsaturated fatty acids (VLCMFAs), mainly in the form of glycosphingolipid and sphingomyelin in the nerve fibers of the white matter and myelin sheath of animal brains. They are important components of biomembranes. Nervonic acid has an important role in medicine and health care, with effects such as promoting brain development, improving memory, regulating blood lipids, and increasing immunity, and can be used to treat neurological disorders such as multiple sclerosis. In addition, studies have shown that nervonic acid has a stimulating effect on the development of the nervous system, in particular, it has an important role in the growth and development of brain nerve cells and optic nerve cells in infants and children.

Currently, the synthesis of very long chain fatty acids such as lignoceric acid (C24:0) and nervonic acid is better understood in plants. Plants use oleic acid as a substrate for carbon chain lengthening through a very long chain fatty acid chain lengthening cycle, which can extend the carbon chain of the original fatty acid by two carbon atoms per cycle. This lengthening process is catalyzed and regulated by a combination of four enzymes (KCS, KCR, HCD and ECR) forming an enzyme complex, of which KCS (3-ketoacyl -CoA synthase) is now generally recognized as the key enzyme that determines the chain length and synthesis rate of ULFAs in plant tissues.

Although nervonic acid has a wide range of uses and is very beneficial to human health, it is difficult to be synthesised by the body itself and needs to be ingested from outside. The current chemical synthesis of nervonic acid is not competitive due to the many by-products, long process routes and low yields, with prices exceeding US$1,000 per kilogram. There is a huge market demand for nervonic acid, but the production of nervonic acid is limited by the slow growth of plants as the main source and the high requirements of the growing environment. Production of nervonic acid by microorganisms holds great promise. However, most of the known nervonic acid-producing microorganisms are pathogenic or have insufficient production potential, making them unavailable for industrial production.

Bio-oil can be used as a renewable energy raw materials, functional food, health care products, special dietary supplements, etc. It is currently mainly obtained from plants and requires a large amount of arable land. Oil-producing microorganisms can be used to synthesise oils from renewable raw materials, which is an important complement to the way oils are obtained. In addition, through synthetic biology methods, the composition of microbial oils can be regulated, showing great promise in functional foods, pharmaceuticals and other fields.

The choice of microorganisms and the efficiency of raw material conversion largely influence the cost of synthesis of oils. In the last decade or so, photosynthetic microorganisms such as algae have received attention to synthesise oils, but despite the possibility of using low-cost carbon dioxide as a carbon source, oil production using microalgae is still difficult to overcome problems such as low biomass, high water consumption and easy pollution, and is costly and difficult to industrialise. Unlike microalgae, oil-producing yeast is a heterotrophic microorganism that can synthesise large amounts of oil from sugars, with oil content accounting for up to 70% of the dry weight of the cells and oil yield exceeding 90 g/L. Moreover, oil-producing yeast is relatively rich in genetic tools and easy to modify, making it an excellent chassis cell for developing special functional fatty acids. Yeast synthesises nervonic acid, the main intracellular form of which is triglyceride. The precursor for nervonic acid synthesis is primarily oleic acid, which, if esterified to the triglyceride form, will no longer be extended to nervonic acid, affecting nervonic acid production. Therefore, screening for ultra-long chain fatty acid esterases is beneficial for the rapid conversion of nervonic acid to triglycerides, pulling the conversion of upstream precursors and thus increasing nervonic acid production.

Therefore, there is an urgent desire in the field to develop fatty acid elongase and esterase with specific substrate preferences to improve the yield of nervonic acid and oils.

### Summary of the invention

The purpose of the present invention is to provide a new method capable of producing high yields of nervonic acid and oils.

In a first aspect of the present invention, it provides an engineered bacteria (strain) for the production of nervonic acid and/or oils, the engineered bacteria (strain) having a gene expression cassette integrated in the genome, the gene expression cassette expressing a protein encoded by a 3-ketoacyl-CoA synthase (KCS) gene and/or an esterase gene.

In another preferred embodiment, the engineered bacteria comprises a yeast.

In another preferred embodiment, the engineered bacteria is selected from the group consisting of: Yarrowia lipolytica, Saccharomyces cerevisiae, methylotrophic yeast, pichia pastoris, Hansenula polymorpha, Kluyveromyces, candida, Cryptococcus magnus, Issatchenkia orientalis, rhodotorula, and a combination thereof.

In another preferred embodiment, the 3-ketoacyl-CoA synthase gene is derived from *Malania oleifera, Cardamine graeca, Acer truncatum, Lunaria annua, Ximenia caffra, Tropaeolum speciosum, Xanthoceras sorbifolia, Brassica, Capsella.*

In another preferred embodiment, the engineered bacteria is *Yarrowia lipolytica,* preferably *Yarrowia lipolytica* Polg-G3-MaoleKCS or *Yarrowia lipolytica* YL-88-B 1.

In another preferred example, the esterase gene is derived from *Malania oleifera, Cardamine graeca, Acer truncatum, Lunaria annua, Ximenia caffra, Tropaeolum speciosum, Xanthoceras sorbifolia, Brassica, Capsella.*

In another preferred embodiment, the esterase gene is selected from the group consisting of: Diacylglycerol acyltransferase (DGAT2), Glycerol-3-phosphate acyltransferase (GPAT) enzyme gene, and a combination thereof.

In another preferred embodiment, the genome is integrated with 1-20 (preferably, 1-10, more preferably, 1-5) of the gene expression cassette.

In a further preferred embodiment, the gene expression cassette is integrated in one or more promoters selected from the group consisting of: TEF, TEFintro, EXP, GPD, GPAT, YAT, hp4d, hp8d, XPR.

In another preferred embodiment, the 3-ketoacyl-CoA synthase (KCS) gene is a codon optimized 3-ketoacyl-CoA synthase (KCS) gene.

In a further preferred embodiment, the esterase gene is a codon-optimized esterase gene.

In a further preferred embodiment, the sequence of the 3-ketoacyl-CoA synthase (KCS) gene is shown in any of SEQ ID NO. 1-11.

In another preferred embodiment, the sequence of the esterase gene is as shown in any one of SEQ ID NO. 48, 49, 50, 51, 52.

In another preferred embodiment, the 3-ketoacyl-CoA synthase (KCS) gene or esterase gene is driven by a constitutive or inducible promoter TEF, TEFintro, EXP, GPD, GPAT, YAT, hp4d, hp8d, XPR.

In a further preferred example, the promoter is selected from the group consisting of: TEF, TEFintro, EXP, GPD, GPAT, YAT, hp4d, hp8d, XPR, and a combination thereof.

In another preferred example, the yield of nervonic acid by engineered bacteria is ≥20 g/L, preferably, 20-30 g/L.

In a further preferred embodiment, the yield of nervonic acid by engineered bacteria is increased by ≥ 25%, preferably, 26-30%.

In another preferred embodiment, the oil yield from shake flask fermentation of the engineered bacteria is ≥ 16 g/L of oil, preferably, 16-18 g/L.

In a further preferred embodiment, the ratio (mass percent) of nervonic acid/total oils of the engineered bacteria is ≥18%, preferably, 18-20%.

In a further preferred embodiment, the fatty acid composition of the oils comprises mainly C16:1, C18:1, C24:1, the percentage by mass of a single component is 15-40%, preferably 18-40%.

In another preferred example, the engineered bacteria have a fermentation biomass (cell dry weight) of ≥ 120 g/L, preferably, 130-200 g/L.

In a further preferred embodiment, the ratio of nervonic acid/total fatty acid (nervonic acid/total fatty acid) of the engineered bacteria is ≥ 20%, preferably 20-65%, more preferably, 25-65%.

In a second aspect of the present invention, it provides a method for producing nervonic acid and/or oil, comprising the steps of:
(i) culturing an engineered bacteria as described in the first aspect of the present invention, thereby obtaining a fermentation product containing nervonic acid and/or oil; and
(ii) isolating the nervonic acid and/or the oil from the fermentation product.

In a third aspect of the present invention, it provides a method for constructing an engineered bacteria as described in the first aspect of the present invention, comprising the steps of:
(a) constructing a vector comprising a gene expression cassette, the expression cassette having the following elements: a selective marker gene, a resistance gene element, an rDNA homologous recombinant gene fragment, and a target gene, the target gene is a 3- ketoacyl -CoA synthase (KCS) gene and/or an esterase gene;
(b) transferring the vector containing the gene expression cassette obtained in step (a) into a recipient strain, thereby obtaining a strain in which the recipient genome is integrated with the gene expression cassette.

In a further preferred embodiment, further comprising step (c): PCR and DNA sequencing to verify the genotype of the strain with the gene expression cassette integrated obtained in step (b); and/or
step (d): fermentation detection for the yields of nervonic acid and/or oils obtained from the strain integrated with gene expression cassettes.

In a further preferred embodiment, the vector in step (a) is a plasmid, a cosmid or a nucleic acid fragment.

In a further preferred embodiment, the expression cassette as described in step (a) further comprises a strong promoter element.

In a further preferred embodiment, the strong promoter element comprises TEFintro, GPAT, EXP, GPD, GPAT, YAT, hp4d, hp8d, XPR.

In a further preferred example, the strong promoter element is TEFintro.

In a further preferred example, the strong promoter element is 1-3, preferably 1-2, more preferably, 2.

In another preferred embodiment, the selective marker gene is selected from the group consisting of: URA, leu, HGR, and a combination thereof.

In a further preferred embodiment, the resistance gene element comprises an ampicillin resistance gene.

In another preferred embodiment, the expression cassette as described in step (a) has the following elements: a strong promoter element TEFintro, a target gene 3-ketoacyl-CoA synthase (KCS) gene and/or an esterase gene, a yeast selective marker gene comprising one or more of URA, leu or HGR, and an ampicillin resistance gene AMP.

In a further preferred example, the number of gene expression cassettes integrated into the receptor genome as described in step (b) is 1-5, preferably 1-3.

In a fourth aspect of the invention, it provides a use of an engineered strain as described in the first aspect of the invention, the engineered strain is used as a strain for fermentative production of nervonic acid and/or oil.

It should be understood that within the scope of the present invention, the above-mentioned technical features of the present invention and the technical features described in detail below (such as embodiments) can be combined with each other to form a new or preferred technical solution. Due to space constraints, I will not repeat them here.

### Description of Drawings

Figure 1 Systematic tree of different 3-ketoacyl-CoA synthase protein sequences.
Figure 2 Sequence comparison of different 3- ketoacyl-CoA synthase proteins. The boxes show the three functionally conserved structural domains of the KCS family "FGNTSSSS", "GSGFKCNSAVW" and "GMGCSA".
Figure 3 Diagnostic PCR validation of the recombinant strains. A: downstream PCR results. CK is the blank control Polg-G3, numbers 1-8 correspond to different transformants of recombinant strains Polg-G3-MaoleKCS398, Po1g-G3-MaoleKCS461 or Po1g-G3-MaoleKCS467. B: Downstream PCR results of different transformants of the recombinant strain Po1g-G3-MaoleKCS817; C: upstream PCR results. CK is the blank control Po1g-G3, numbers 1-8 correspond to the different transformants of recombinant strains Po1g-G3-MaoleKCS398, Po1g-G3-MaoleKCS461, Po1g-G3-MaoleKCS467 or Po1g-G3-MaoleKCS817.
Figure 4 Shake flask fermentation results of the recombinant strains. Comparison of dry weight, total lipid yield and total lipid content between strains. strainCK: control strain Po1g-G3; strain398: Po1g-G3-MaoleKCS398; strain461: Po1g-G3-MaoleKCS461; strain467: Po1g-G3-MaoleKCS467 strain817: Polg-G3-MaoleKCS817.
Figure 5 Results of fatty acid gas phase analysis of recombinant strains. CK: control strain Po1g-G3; MaoleKCS: Po1g-G3-MaoleKCS398. The numbers 1-11 represent the different fatty acid compositions, in the order of C16:0, C16:1, C18:0, C18:1, C18:2, C20:0, C20:1, C22:0, C22:1, C24:0 and C24:1.
Figure 6 Results of shake flask fermentation of recombinant strains overexpressing MaoleKCS. A: comparison of dry weight, total lipid yield and total lipid content between different strains. B: analysis of fatty acid composition of different strains. strainCK: control strain Po1g-G3-MaoleKCS; strain#1: Polg-G3-3MaoleKCS39S; strain#2: Polg-G3-3MaoleKCS461; strain#3: Po1g-G3-3MaoleKCS467; strain#4: Po1g-G3-3MaoleKCS817.
Figure 7 Gas phase analysis graph of the fatty acid composition of the recombinant strains overexpressing MaoleKCS. a: control strain Po1g-G3-MaoleKCS; b: Po1g-G3-3MaoleKCS398; c: Po1g-G3-3MaoleKCS461; d: Po1g-G3-3MaoleKCS467; e: Po1g-G3-3MaoleKCS817. Numbers 1-11 represent the different fatty acid components, in the order of C16:0, C16:1, C18:0, C18:1, C18:2, C20:0, C20:1, C22:0, C22:1, C24:0 and C24:1.
Figure 8 Fermenter fermentation results for the recombinant strains.
Figure 9 Gas phase analysis graph of the fatty acid composition of the recombinant strains fermented in the fermenter for 168 h. Numbers 1-11 represent the different fatty acid compositions in the order of C16: 0, C16: 1, C18: 0, C18:1, C18:2, C20:0, C20:1, C22:0, C22:1, C24:0 and C24:1.
Figure 10 shows the oil yield analysis of the recombinant strain of esterase.
Figure 11 shows the analysis of nervonic acid production of the esterase recombinant strain.
Figure 12 shows the change in fatty acid composition of the esterase recombinant strain.
Figure 13 shows the positive clone identification results of the esterase recombinant strains.

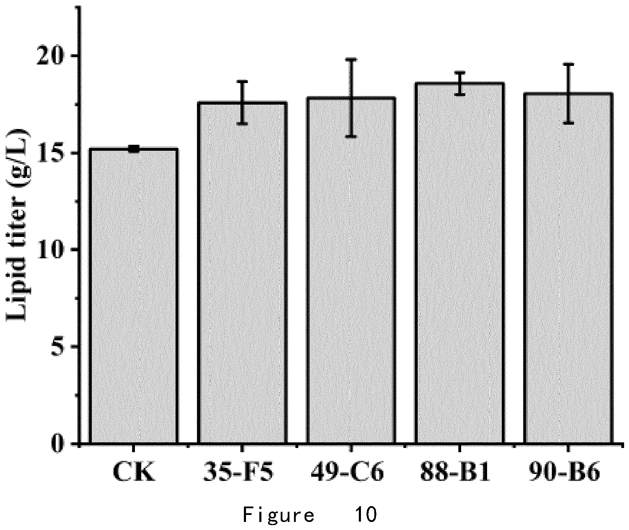

Wherein in Figures 10-12, CK is Polg-G3-CgKCS.

### DETAILED DESCRIPTION

After extensive and intensive research, the present inventors have accidentally constructed for the first time a yeast engineered strain with a gene expression cassette integrated in the genome of the engineered strain, the gene expression cassette expressing proteins encoded by the 3- ketoacyl -CoA synthase (KCS) gene and/or the esterase gene, the engineered strain of the present invention significantly increases the yield of nervonic acid and/or oil and the engineered strain of the present invention also alters the the fatty acid composition of the oil. On this basis, the present invention is completed

### Terms

Unless otherwise defined, all technical and scientific terms used herein have the same meaning as commonly understood by those of ordinary skill in the art of the invention.

As used herein, when used with reference to a specifically enumerated value, the term "about" means that the value can vary from the enumerated value by no more than 1%. For example, as used herein, the expression "about 100" includes 99 and 101 and all values between 99 and 101 (e.g., 99.1, 99.2, 99.3, 99.4, etc.).

As used herein, the terms "contains" or "includes (comprises)" may be open-ended, semi-closed, and closed-ended. In other words, the term also includes "consisting essentially of", or "consisting of".

As used herein, the terms "above" and "below" include this number, e.g., "more than 20%" means ≥ 20%, "more than 8:1 " means ≥ 8:1.

### Starting strain

As used herein, the terms "starting strain of the invention" or "starting microorganism of the invention" are used interchangeably and both refer to the *Yarrowia lipolytica Y. lipolytica* Polg (MATa, leu2-270, ura3-302::URA3, xpr2-3) and *Y. lipolytica* Po1g-KCS (MATa, leu2-270, ura3-302::URA3, xpr2-3, KCS), wherein *Y. lipolytica* Po1g was purchased from Yeastern Biotech Co., Ltd, *Y. lipolytica* Po1g-KCS (obtained from Qingdao Institute of Bioenergy and Processes, Chinese Academy of Sciences).

It should be understood that the starting strain includes not only the *Yarrowia lipolytica Y. lipolytica* Po1g of the present invention, but also its derivative strains.

### Nervonic acid

Nervonic acid (*cis*-15-Tetracosenoic acid, 24: 1Δ15) is a very long chain monounsaturated fatty acids (VLCMFAs), mainly in the form of glycosphingolipid and sphingomyelin in the nerve fibers of the white matter and myelin sheath of the animal brain, which are important components of biological membranes. Nervonic acid has an important role in medicine and health care, with effects such as promoting brain development, improving memory, regulating blood lipids, and enhancing immunity, and can be used to treat neurological disorders such as multiple sclerosis. In addition, studies have shown that nervonic acid has a stimulating effect on the development of the nervous system, especially it has an important role in the growth and development of brain nerve cells and optic nerve cells in infants and children.

### Oils

Microbial oils have important applications in bioenergy, food and medicine. *Yarrowia lipolytica* is an exclusively aerobic oil-producing yeast that accumulates large amounts of neutral lipids, the most important components of which include palmitoleic acid and oleic acid, the sum of the two contents through the enhanced oil production pathway accounts for more than 70% of the total fatty acid ratio. Palmitoleic acid has therapeutic effects in some chronic diseases such as metabolic syndrome, diabetes and inflammation, and palmitoleic acid can inhibit melanin factors in human cells and improve skin pigmentation. Oleic acid is a cis-structure that is useful in softening blood vessels and plays an important role in the metabolic processes of humans and animals, health benefits of consuming cooking oils with high oleic acid content. In addition, oleic acid is also an important raw material for paint drier, lubricant thickener, wool spinning industry, printing and dyeing industry. The mixed oil synthesized by oil-producing yeast can be used as raw material for biodiesel refining.

### 3- ketoacyl -CoA synthase (KCS) gene

In the present invention, the protein product encoded by the "3- ketoacyl -CoA synthase (KCS) gene" described herein is 3-ketoacyl-CoA synthase, which is involved in catalyzing the synthesis of nervonic acid.

The 3-ketoacyl-CoA synthase (KCS) gene encodes a protein product of 3-ketoacyl-CoA synthase, which catalyzes the final synthesis of nervonic acid. 3-ketoacyl-CoA synthase (KCS) contains a single open frame, encoding a protein of 512-653 amino acids.

After extensive creative experiments, the present invention has found that expression cassettes integrating expression of the 3-ketoacyl-CoA synthase (KCS) gene in the engineered strain can significantly increase the yield of nervonic acid and reduce the content of unsaturated fatty acids. Therefore, the modified engineered strains provided by the technical solution have broad practical application value and prospects.

The person skilled in the art can obtain the sequence of the 3-ketoacyl-CoA synthase (KCS) gene using common methods, such as from NCBI.

The 3-ketoacyl-CoA synthase (KCS) gene of the present invention also includes 3-ketoacyl-CoA synthase (KCS) genes from different sources, different homologous sequences from the same source, and codon-optimized 3-ketoacyl -CoA synthase (KCS) genes.

### Esterases

In the present invention, the "esterase genes" described herein include Diacylglycerol acyltransferase (DGAT2) and Glycerol-3-phosphate acyltransferase (GPAT) genes, encoding protein products of Diacylglycerol acyltransferase (DGAT2) and Glycerol-3-phosphate acyltransferase (GPAT), Diacylglycerol acyltransferase is involved in catalyzing triacylglycerol, and Glycerol-3-phosphate acyltransferase is involved in catalyzing the synthesis of lysophosphatidic acid.

Diacylglycerol acyltransferase (DGAT2) contains MoDGAT2 (Maole_015949), MoDGAT2 (Maole_010035), encoding a protein of 403-522 amino acids.

Glycerol-3-phosphate acyltransferase (GPAT) enzyme gene contains MoGPAT (Maole_006088), MoGPAT (Maole_006089), and MoGPAT (Maole_006090), encoding proteins of 231-370 amino acids.

The present invention, after extensive creative experiments, has found that expression cassettes integrating expression of the 3-ketoacyl-CoA synthase (KCS) gene and/or esterase in the engineered strain can significantly increase the yield of nervonic acid or oil, reduce the content of unsaturated fatty acids, change the fatty acid composition of oil, increase the proportion of oleic acid to total oil, increase the proportion of nervonic acid to total oil, increase the fermentation concentration of nervonic acid. Therefore, the modified engineered strains provided by the technical solution of the present invention have broad practical application value and prospects.

The person skilled in the art can obtain the sequence of the esterase gene using a common method, such as from NCBI.

The esterase genes of the present invention also include esterase genes from different sources, different homologous sequences of the same source.

### Primers

As used herein, the term "primer" refers to a generic term for an oligonucleotide that can be paired with a template and used as a starting point for the synthesis of a DNA strand complementary to the template in the presence of DNA polymerase. Primers can be natural RNA, DNA, or any form of natural nucleotide. Primers can even be non-natural nucleotides such as LNA or ZNA.

A primer is "roughly" (or "essentially") complementary to a specific sequence on one strand of the template. The primer must be fully complementary to a strand on the template to start the extension, but the sequence of the primer does not have to be fully complementary to the sequence of the template. For example, adding a segment of sequence that is not complementary to the template to the 5' end of a primer that is complementary to the template at the 3' end would still be roughly complementary to the template. Primers that are not fully complementary can also form primer-template complexes with the template for amplification as long as there are primers long enough to bind adequately to the template.

### Construction of engineered strains

The present invention provides a method for constructing a high yielding strain of genetically engineered bacteria nervonic acid, comprising (but not limited to) the steps of:
1. Constructing a vector containing a gene expression cassette

Synthesize the gene after codon optimization, design PCR primers for the 3 - ketoacyl-CoA synthase (KCS) gene, clone to obtain the target gene, and construct the plasmid by Gibson assembly seamless ligation method. 2. The obtained vector containing the gene expression cassette is transferred into the recipient strain to obtain a strain in which the recipient genome is integrated with the gene expression cassette. In a preferred example, the constructed vector with the expression cassette is transferred into a starting strain by intergeneric conjugal transfer, and positive zygotes are selected under certain resistance screening conditions.

In a preferred embodiment, it further comprises step 3: PCR was performed to verify the genotype of the strain that incorporates the gene expression cassette obtained in step 2; and/or step 4: fermentation to detect the yield of nervonic acid of the strain that incorporates the gene expression cassette obtained. In a preferred example, positive zygotes with certain resistance are cultured in liquid medium and total DNA of the bacterium is extracted for PCR validation of the genotype. Multiple primer pairs can be designed for the target gene to verify the integration of the corresponding gene into the target genome. The correct size base fragment obtained by PCR amplification is recovered and the correct genetically engineered strain is finally confirmed by DNA sequencing.

The strain obtained by the present invention, the Yarrowia lipolytica strain Po1g-G3 - MaoleKCS, has a significantly improved ability to produce nervonic acid by fermentation.

In a preferred embodiment, the present invention uses technical solutions as:
(1) Screening and analysis of malania oleifera 3-ketoacyl-CoA synthase by bioinformatics analysis.
(2) Using Yarrowia lipolytica as a starting strain, the gene for malania oleifera 3- ketoacyl-CoA synthase (see SEQ ID No. 1-11) was introduced to enable or enhance the ability of Yarrowia lipolytica yeast to synthesize nervonic acid.

Further, using the Yarrowia lipolytica yeast Po1g-G3 as a starting strain, the MaoleKCS gene was introduced by homologous recombination to obtain the strain Polg-G3-MaoleKCS.

Still further, the homologous recombinant plasmid pYLEX-rDNA-MaoleKCS-URA was constructed first, then the resulting plasmid was enzymatically cleaved and transformed into the Yarrowia lipolytica yeast strain Polg-G3-ΔURA, and the recombinant strain Po1g-G3-MaoleKCS with the introduced MaoleKCS gene is obtained by screening the transformants through uracil nutrient-deficient plates.

Further, the homologous recombinant plasmid pYLEX-rDNA-MaoleKCS-URA uses plasmid pYLEX-URA as a backbone template, and the MaoleKCS expression cassette is upstream and downstream of two fragments of the Yarrowia lipolytica 26S rDNA, respectively (see SEQ ID No. 12 and SEQ ID No. 13).

(3) Overexpression of the malania oleifera 3-ketoacyl-CoA synthase gene (see SEQ ID No. 1-11), using Yarrowia lipolytica as the starting strain, enabling or enhancing the ability of Yarrowia lipolytica to synthesize nervonic acid.

Further, using the Yarrowia lipolytica Po1g-G3-MaoleKCS as the starting strain, the MaoleKCS gene was introduced by homologous recombination to obtain strain Po1g-G3-3MaoleKCS.

Further, the homologous recombinant plasmid pYLEX-FAD2-MaoleKCS-URA was constructed first, and then the resulting plasmid was enzymatically cleaved and transformed into the Yarrowia lipolytica strain Polg-G3-MaoleKCS-ΔURA, and the recombinant strain Po1g-G3-3MaoleKCS, which introduces the MaoleKCS gene, was obtained by screening transformants through uracil nutrient-deficient plates.

Further, the homologous recombinant plasmid pYLEX-FAD2-MaoleKCS-URA uses plasmid pYLEX-URA as a backbone template, and the MaoleKCS expression cassette is upstream and downstream of two fragments of the Yarrowia lipolytica FAD2 gene, respectively (see SEQ ID No. 14 and SEQ ID No. 15).

(4) The above recombinant strain was subjected to fermenter culture, and the proportion of nervonic acid to total fatty acid can reach 36.9%, and the yield of nervonic acid reaches 25.7 g/L.

The present invention, while providing a method for constructing a genetically engineered strain of nervonic acid, also provides a method for constructing a high yielding strain of oil, comprising (but not limited to) the steps of:
1 . constructing a vector containing a gene expression cassette

Synthesizing the gene after codon optimization, designing PCR primers for Diacylglycerol acyltransferase (DGAT2) and Glycerol-3-phosphate acyltransferase (GPAT) enzyme genes, cloning to obtain the target gene, and constructing a plasmid by the Gibson assembly seamless ligation method.

2 . The obtained vector containing the gene expression cassette is transferred into the recipient strain to obtain a strain with the recipient genome integrated with the gene expression cassette. In a preferred embodiment, the constructed vector with the expression cassette is transferred into the starting strain by intergeneric conjugal transfer and the positive zygotes are selected under certain resistance screening conditions.

In a preferred embodiment, it further comprises step 3: PCR was performed to verify the genotype of the strain incorporating the gene expression cassette obtained in step 2; and/or step 4: fermentation to detect the yield of nervonic acid of the strain that incorporates the gene expression cassette obtained. In a preferred example, positive zygotes with certain resistance are cultured in liquid medium and total DNA of the bacterium is extracted for PCR validation of the genotype. Multiple primer pairs can be designed for the target gene to verify the integration of the corresponding gene into the target genome. The correct size base fragment obtained by PCR amplification is recovered and the correct genetically engineered strain is finally confirmed by DNA sequencing.

The strain obtained by the present invention, the Yarrowia lipolytica strain YL-88-B1, has a significantly improved ability to produce oil and nervonic acid by fermentation.

In another preferred embodiment, the present invention uses the technical solutions of:
(1) Screening and analysis of Diacylglycerol acyltransferase (DGAT2) and Glycerol-3-phosphate acyltransferase (GPAT) by bioinformatics analysis.
(2) Using the Yarrowia lipolytica strain Polg-G3-CgKCS, which already expresses the fatty acid elongase CgKCS, as a starting strain, the Diacylglycerol acyltransferase (DGAT2) and Glycerol-3-phosphate acyltransferase (GPAT) enzymes from malania oleifera (see SEQ ID No. 48, 49, 50, 51, 52) were introduced to enable or enhance the ability of Yarrowia lipolytica to synthesise oil and nervonic acid.

Further, using the Yarrowia lipolytica Po1g-G3-CgKCS as a starting strain, the genes for Diacylglycerol acyltransferase (DGAT2) and Glycerol-3-phosphate acyltransferase (GPAT) enzymes were introduced by homologous recombination to obtain strains YL-35-F5, YL-49-C6, YL-88-B1 and YL-90-B6 and when the elongase CgKCS and esterase were expressed by the method of the present invention, the oil yield of strain YL-88-B1 at the shake flask fermentation level is increased by 22.1% and the proportion of nervonic acid to total fatty acids is up to 18-20%.

### Fermentation for the production of nervonic acid

The genetically modified strain of the present invention can be used for the fermentation production of nervonic acid and in a preferred example, the medium can comprise, but is not limited to, the following weight-to-volume ratios in terms of total volume of medium:
YPD liquid medium: glucose 20 g/L, peptone 20 g/L, yeast extract 10 g/L, 1.5% agar is added if solid medium is required; for routine culture.

Uracil nutrient-deficient medium (YNB-Δura): YNB (Yeast nitrogen base without amino acids and ammonium) 1.7 g/L, glucose 20.0 g/L, CSM-Ura (MP Biomedicals) 0.67 g/L, ammonium sulfate 5 g/L, if solid medium is required add 1.5% agar; for the screening and activation culture of recombinant strains.

Shake flask fermentation medium: glucose 150 g/L, yeast extract 6 g/L, ammonium sulfate 12 g/L.

The incubation temperature used for E. coli was 37°C and the speed of the shaker was 200 rpm for liquid culture, and the medium used was as follows:
LB medium: tryptone 10 g/L, yeast extract 5 g/L, sodium chloride 10 g/L, if solid medium is required add 1.5% agar; used for routine culture.

Amp resistance medium: LB medium with high concentration of Amp solution to give a final concentration of 100 µg/ml of Amp, 1.5% agar is added if solid medium is required; for screening and expansion of recombinant bacterial strains.

The fermentation product of the strain of the invention can be used to prepare nervonic acid.

### Pharmaceutical compositions and methods of administration

The nervonic acid in the fermentation product of the strain of the invention can be used to prepare drugs. The nervonic acid and oil of the present invention can be administered to mammals (e.g. humans) and can be administered orally, rectally, parenterally (intravenously, intramuscularly or subcutaneously), topically, etc. Said nervonic acid and oil may be administered alone or in combination with other pharmaceutically acceptable compounds. It is to be noted that the nervonic acid and oil of the present invention can be administered in combination.

The solid dosage forms for oral administration include capsules, tablets, pills, powders and granules. In these solid dosage forms, the active compound is mixed with at least one conventional inert excipient (or carrier), such as sodium citrate or dicalcium phosphate, or with (a) fillers or bulking agents, for example, starch, lactose, sucrose, glucose, mannitol and silicic acid; (b) binders, for example, hydroxymethyl cellulose, alginate, gelatin, polyvinylpyrrolidone, sucrose and arabic gum; (c) wetting agent, e.g. glycerol; (d) disintegrating agents, e.g. agar, calcium carbonate, potato starch or tapioca, alginic acid, certain complex silicates, and sodium carbonate; (e) retarding solvents, e.g. paraffin; (f) Absorption accelerators, e.g. quaternary ammonium compounds; (g) Wetting agents, e.g. cetanol andglycerin monostearate; (h) adsorbents, e.g. kaolin; and (i) Lubricants, e.g. , talc, calcium stearate, magnesium stearate, solid polyethylene glycol, lauryl sodium sulfate, or mixtures thereof. In capsules, tablets and pills, the dosage form may also contain a buffering agent.

Solid dosage forms such as tablets, sugar pills, capsules, pills and granules may be prepared using coatings and shell materials, such as enteric coatings and other materials well known in the art. They may contain opacifying agents and the release of the active compound or compounds in such compositions may be released in a delayed manner in a portion of the digestive tract. Examples of embedding components that may be used are polymeric substances and wax-like substances. If necessary, the active compound may also be formed in microcapsule form with one or more of the above excipients.

Liquid dosage forms for oral administration include pharmaceutically acceptable emulsions, solutions, suspensions, syrups or tinctures. In addition to the active compound, the liquid dosage form may contain inert diluents routinely employed in the art, such as water or other solvents, solubilizers and emulsifiers, for example, ethanol, isopropanol, ethyl carbonate, ethyl acetate, propylene glycol, 1,3 -butylene glycol, dimethyl formamide and oils, in particular cottonseed oil, peanut oil, corn germ oil, olive oil, castor oil and sesame oil, or mixtures of these substances.

In addition to these inert diluents, the compositions may also contain adjuvants such as wetting agents, emulsifiers and suspending agents, sweeteners and fragrances.

In addition to the active compound, the suspension may comprise suspending agents such as, for example, ethoxylated octadecanol, polyoxyethylene sorbitol and Isosorbide Dinitrate, microcrystalline cellulose, aluminum methoxide and agar or mixtures of these substances.

Compositions for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents or excipients include water, ethanol, polyhydric alcohols and suitable mixtures thereof.

Dosage forms of the compounds of the invention for topical administration include ointments, powders, patches, sprays and inhalations. The active ingredient is mixed under sterile conditions with a physiologically acceptable carrier and any preservatives, buffers, or propellants that may be required if necessary.

The pharmaceutical composition is used when a safe and effective amount of the nervonic acid and oil of the invention is applied to a mammal (e.g. human) in need of treatment, wherein the dose at the time of administration is the pharmacologically considered effective dose for administration, which for an individual of 60 kg body weight is usually 1 to 1000 mg per day, preferably 20 to 500 mg. Of course, the specific dose should also take into account the route of administration, the health status of the individual and other factors, which are within the skill of the skilled practitioner.

### The main advantages of the present invention include:

(1) The invention significantly increases the production of nervonic acid by expressing the 3-ketoacyl-CoA synthase (KCS) gene.
(2) Effective expression of a single enzyme of the present invention enables engineered strains of yeast, such as Yarrowia lipolytica, to have or enhance the ability to synthesize nervonic acid.
(3) The proportion of nervonic acid to total fatty acids in the cells of the recombinant strain of the present invention can reach 36.9%, and the yield of nervonic acid can reach 25.7 g/L. The malania oleifera 3-ketoacyl-CoA synthase provided by the present invention can be used to construct high-yielding nervonic acid of yeast engineering strains, which has a wide range of application prospects in chemical, pharmaceutical and health care and other fields.
(4) By expressing esterase alone or co-expressing esterase and ketoacyl-CoA synthase (KCS) genes, the present invention has significantly increased the yield of nervonic acid and oil, changed the fatty acid composition of oil, increased the proportion of oleic acid to total oil, increased the proportion of nervonic acid to total oil, and increased the fermentation concentration of nervonic acid.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Percentages and parts are percentages by weight and parts by weight unless otherwise stated.

Materials and reagents in embodiments of the present invention are commercially available products if not otherwise specified.

### Example 1: Synthesis of nervonic acid by yeast expressing MaoleKCS

### 1. Materials and methods

### 1.1 Strain and culture conditions

The starting strain of Yarrowia lipolytica, *Y. lipolytica* Po1g (MATa, leu2-270, ura3-302::URA3, xpr2-3) used in the example was purchased from Yeastern Biotech Co., Ltd. The strain Polg-G3-ΔURA was obtained by the following method. Specifically, strain Po1g-G3 was obtained by overexpressing the endogenous genes DGAT1, ACC1, and SCD genes of Yarrowia lipolytica (Qiao and Stephanopoulos. Metabolic Engineering, 2015, 29:56-65), and then the uracil nutrient-deficient strain Polg-G3-ΔURA was then obtained by reverse screening with the 5-FOA method.

The culture temperature used for the Yarrowia lipolytica was 28°C, the shaker speed was 220 rpm for liquid culture, and the medium used was as follows:
YPD liquid medium: glucose 20 g/L, peptone 20 g/L, yeast extract 10 g/L, or 1.5% agar is added if solid medium is required; used for routine culture.

Uracil nutrient-deficient medium (YNB-Δura): YNB (Yeast nitrogen base without amino acids and ammonium) 1.7 g/L, glucose 20.0 g/L, CSM-Ura (MP Biomedicals) 0.67 g/L, ammonium sulfate 5 g/L, 1.5% agar is added if solid medium is required; used for screening and activation culture of recombinant strains.

Shake flask fermentation medium: glucose 150 g/L, yeast extract 6 g/L, ammonium sulfate 12 g/L.

The culture temperature used for E. coli was 37°C, and the shaker speed was 200 rpm for liquid culture, and the medium used was as follows:
LB medium: tryptone 10 g/L, yeast extract 5 g/L, sodium chloride 10 g/L, if solid medium is needed then add 1.5% agar; used for regular culture.

Amp resistance medium: LB medium with high concentration of Amp solution to make the final concentration of Amp 100 µg/ml, add 1.5% agar if solid medium is needed; used for screening and expansion culture of recombinant bacterial strains.

### 1.2 Screening and expression of malania oleifera 3-ketoacyl-CoA synthase gene

### 1.2.1 Bioinformatic analysis of malania oleifera 3-ketoacyl-CoA synthase gene

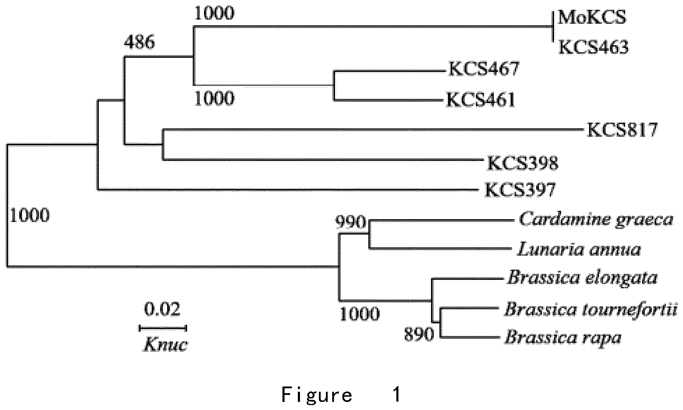

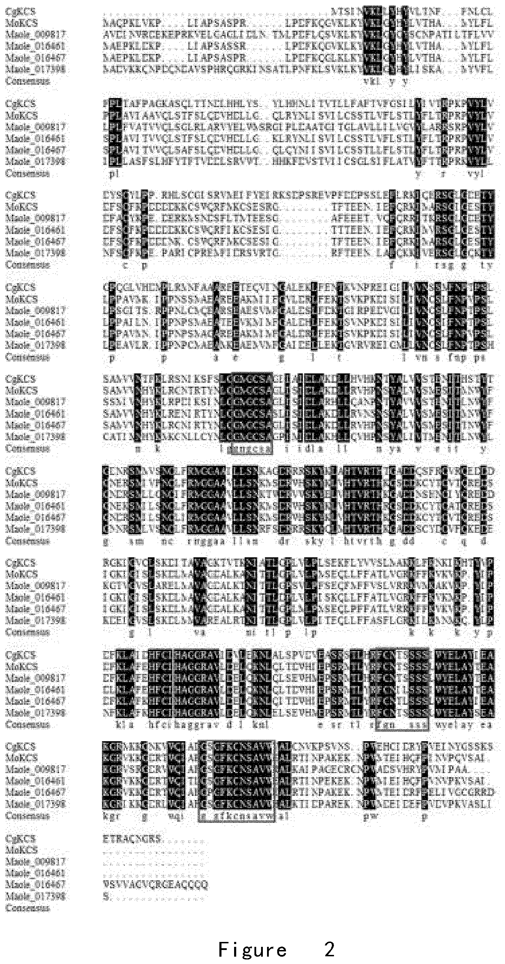

Twelve potential malania oleifera 3-ketoacyl-CoA synthase genes were obtained from the literature (Xu, et al. GigaScience, 2019, 8:1-14) genomic data, namely Maole_002411, Maole_003085, Maole_004215, Maole_ 005654, Maole_008020, Maole_009817, Maole_016461, Maole_016463, Maole_016466, Maole_016467, Maole_017397 and Maole_017398, respectively. six of these genes were then selected and named as KCS397 (Maole_017397), KCS398 (Maole_017398), KCS461 (Maole_016461), KCS463 (Maole_016463), KCS467 (Maole_016467) and KCS817 (Maole_009817), respectively and the corresponding amino acid sequences were obtained, in the order of Maole_017397.T1, Maole_017398.T1, Maole_016461.T1, Maole_016463.T1, Maole_016467.T1 and Maole_009817.T1, as shown in Table 1. Search the NCBI database for MoKCS (GenBank: QDA34238.1, from M. oleifera), CgKCS (GenBank: ACJ61778.1, from C. graeca), LaKCS (GenBank:EU871787.1, from Lunaria annua), BrKCS (GenBank: GU325723, from Brassica rapa), BtKCS (GenBank:KF664165, from Brassica tournefortii), BeKCS (GenBank:KF664168, from Brassica elongata) that has been identified as a 3- ketoacyl-CoA synthase, and their gene and amino acid sequences. After the amino acid sequences of KCS397, KCS398, KCS461, KCS463, KCS467, KCS817, MoKCS, CgKCS, LaKCS, BrKCS, BtKCS, and BeKCS were subjected to protein sequence multiple alignment using the self-contained ClusterW in MEGA5.1, the Neighbor -joining algorithm (self-checked 1000 times), a phylogenetic tree was drawn, see Figure 1. 4 of KCS (KCS398, KCS461, KCS467 and KCS817) genes were selected and the sequences of CgKCS, MoKCS and the four selected KCS proteins were compared using NCBI online structural domain analysis software (https://www.ncbi.nlm.nih.gov/ Structure/cdd/wrpsb.cgi) and DNAMAN software, see Figure 2.

### 1.2.2 Vector construction

The KCS398, KCS461, KCS467 and KCS817 genes were synthesised by Wuxi Qinglan Biotechnology Co., Ltd. after codon optimization (the sequences of the optimized genes are SEQ ID No. 2, 3, 5 and 6 in order) to obtain a shuttle plasmid with the MaoleKCS gene and the ampicillin resistance gene, named pMv-MaoleKCS#-AMP (Note: # represents different MaoleKCS gene numbers, i.e. 398, 461, 467 or 817, and the same applies to the back).

The plasmid vector was constructed using the Gibson assembly method and all assembly fragments were obtained by PCR amplification. The plasmid backbone was derived from the pYLEX plasmid (Yeastern Biotech Co., Ltd). All PCR amplifications were carried out using KAPA HiFi high-fidelity thermostable DNA polymerase in a 50 µl amplification system (2 × KAPA Mix, 25 ul; 1.5 ul for each of 10 µM primer; 1 µl of template; fill to 50 µl with water) to obtain each of the DNA sequences. The amplification conditions are: pre-denaturation at 95°C for 3 min, denaturation at 98°C for 20 sec, annealing at 60-72°C for 15 sec, extension at 72°C, extension times are calculated at 30 seconds per kb for 29-35 cycles and extension at 72°C for 10 min.

**Table 1. malania oleifera and 3-ketoacyl-CoA synthase screened from the database**

| Enzyme No. | Gene ID | SEQ ID NO.: |
|---|---|---|
| KCS397 | Maole_017397 | 1 |
| KCS398 | Maole_017398 | 2 |
| KCS461 | Maole_016461 | 3 |
| KCS463 | Maole_016463 | 4 |
| KCS467 | Maole_016467 | 5 |
| KCS817 | Maole_009817 | 6 |
| BrKCS | GU325723 | 7 |
| BtKCS | KF664165 | 8 |
| BeKCS | KF664168 | 9 |
| CgKCS | EU871788 | 10 |
| LaKCS | EU871787.1 | 11 |

The plasmid pYLEX-URA was obtained by replacing the LEU2 gene on the pYLEX plasmid with the URA3 gene using the Gibson assembly method; the pYLEX-URA was then used as the backbone to construct the homologous recombinant plasmid pYLEX-rDNA-CgKCS-URA (CgKCS is the KCS gene of Cardamine in 1.2), in which the CgKCS expression cassette was upstream and downstream of the partial sequence of the 26S rDNA of *Yarrowia lipolytica* (see SEQ ID No. 12 and SEQ ID No. 13) to obtain a homologous recombinant plasmid with a targeted integration site in the 26S rDNA region. The plasmid pYLEX-rDNA-CgKCS-URA was used as a backbone to construct the homologous recombinant plasmid pYLEX-rDNA-MaoleKCS-URA by replacing the CgKCS gene with the MaoleKCS gene using the Gibson assembly method.

The specific construction process was as follows:
Plasmid pYLEX-rDNA-CgKCS-URA was obtained directly from the patent applicant's laboratory. The backbone DNA fragments with AMP and URA screening markers were first amplified using P#-F and P#-R primers using the pYLEX-rDNA-CgKCS-URA plasmid as template; then the MaoleKCS gene fragment was then amplified using pMv#-F and pMv#-R primers, using the plasmid pMv-MaoleKCS#-AMP with the MaoleKCS gene as a template, respectively; transformation of E. coli competent Trans-T1 after Gibson assembly of the backbone and MaoleKCS gene fragment (Beijing All-Style Gold Biotechnology Co. Ltd.), and then the homologous recombinant plasmid pYLEX-rDNA-MaoleKCS#-URA was obtained after colony PCR and sequencing verification with primers rDNA-SF and rDNA-SR (see Table 2 for the primers).

**Table 2. Primers used for plasmid construction and colony PCR**

| Primer | Sequence(5'→3') | SEQ ID NO.: |
|---|---|---|
| P398-F | ATTTCTTAACCCCACGTTGCCGG | 16 |
| P398-R | CCAGCATCTGCGGTTAGTACTGCAAAAAGT | 17 |
| pMv398-F | ACCGCAGATGCTGGAACTGCAGGAGTGG | 18 |
| pMv398-R | CAACGTGGGGTTAAGAAATCAGAGAAGCC ACTTTGGGCAC | 19 |
| P467-F | AGAAGATAACCCCACGTTGCCGG | 20 |
| P467-R | CGGCCATCTGCGGTTAGTACTGCAAAAAGT | 21 |
| pMv467-F | CCGCAGATGGCCGAGCCCAAGCT | 22 |
| pMv467-R | AACGTGGGGTTATCTTCTGCCGCCCTGTTCC C | 23 |
| P461-F | CCATTTAACCCCACGTTGCCGGT | 24 |
| P461-R | CAGCCATCTGCGGTTAGTACTGCAAAAAGT | 25 |
| pMv461-F | ACCGCAGATGGCTGAGCCCAAGCTG | 26 |
| pMv461-R | AACGTGGGGTTAAATGGCAGACACTTTGGG CACG | 27 |
| P817-F | CGCTTAACCCCACGTTGCCG | 28 |
| P817-R | CGTGCATCTGCGGTTAGTACTGCAAAAAGT | 29 |
| pMv817-F | CCGCAGATGCACGCCGCCATCTCT | 30 |
| pMv817-R | CGTGGGGTTAAGCGGCGGGGATCATAACG | 31 |
| rDNA-SF | TGTGGTTGGGACTTTAGCCA | 32 |
| rDNA-SR | AATAAGAGCCTCGACCTGGG | 33 |
| ylr-duf | TGTGACTTGCAGATGTGAATCA | 34 |
| ylr-dur | CTTAACGGCAAGTGTGGGAA | 35 |
| ylr-ddf | CACCTTGTACTGACAGTCACA | 36 |
| ylr-ddr | CAACAAGTCCATCAGTAGGG | 37 |

### 1.2.3 Yeast transformation

The recombinant plasmid pYLEX-rDNA-MaoleKCS-URA was digested using *Not* I restriction endonuclease, confirmed by agarose gel electrophoresis, transformation of *Yarrowia lipolytica* Polg-G3-ΔURA using LiAc transformation after recovery of fragment gels for transformation, the transformation system is shown in Table 3 and the method steps are described as follows:

**Table 3 Yarrowia lipolytica transformation systems**

| reactant | dosage |
|---|---|
| 50% PEG (3350) | 90 µL |
| 2M LiAc | 5 µL |
| 2M DTT (Ready-to-Use) | 5 µL |
| Salmon liner DNA | 2.5 µL |
| Liner plasmid | 1.0 µg |
| *Y. lipolytica* cells | 3 loops |
| DMSO | 2 µL |

(1) Add the components according to the transformation system in Table 3 and mix well;
(2) Vortex shaking after a water bath at 30°C for 1 h and heat shock at 39°C for 10 min;
(3) The mixture of the transformation system was directly applied to the screening plate of YNB-Δura medium and incubated at 28°C for 2-4 days to obtain the recombinant strain with the introduced MaoleKCS gene, named Polg-G3-MaoleKCS#.

### 1.2.4 Diagnostic PCR validation of the recombinant strains

Diagnostic PCR validation was performed on different transformants of each recombinant strain obtained above.

Single colonies of the recombinant strains were picked from the screening plates and grown on YPD plates for about 24 h. Pick the bacterial cell and the genomic DNA of the recombinant strains was extracted using Rapid Fungi Genomic DNA Isolation Kit (Sango Biotech) and the genomic DNA was used as a template for PCR verification of the upstream part of the introduced gene MaoleKCS by primers ylr-duf and ylr-dur. The downstream part was verified by primers ylr-ddf and ylr-ddr (see Table 1 for the primers).

PCR amplification was performed using EasyTaq DNA polymerase and the amplification systems were all 20 µl (2× EasyTaq Mix, 10 µl; 0.6 µl each of 10 µM primers; 1 µl of template; water was added to make up to 20 µl) and the amplified fragments were determined by agarose gel electrophoresis. The amplification conditions were: pre-denaturation at 94°C for 3 minutes; denaturation at 94°C for 30 seconds, annealing at 55°C for 30 seconds and extension at 72°C, with an extension time calculated at 60 seconds per kb and a cycle number of 36; extension at 72°C for 10 minutes.

### 1.2.5 Shake flask fermentation culture of recombinant strains

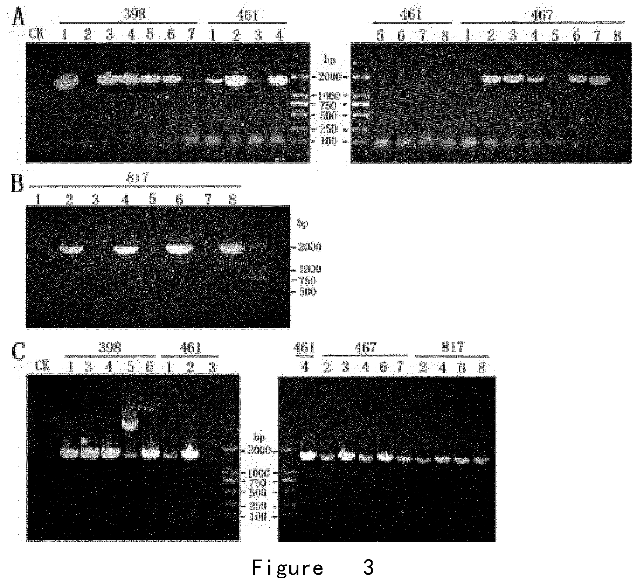

The recombinant strain, which was successfully validated both upstream and downstream (see Figure 3), was inoculated in 20 ml YPD medium in a 250 ml conical flask), grown for approximately 24 h and transferred to a YPD medium plate, grown for approximately 24 h and inoculated into 5 ml YNB-Δura medium (50 ml conical flask), grown for 36 h and 100 µl was aspirated and transferred to a fresh 5 ml YNB-Δ ura medium for 36 hours and then transferred to 30 ml shake flask fermentation medium to give an initial OD uniformity of 0.08, three parallels per group, cultured for 168 hours. The dry weight of the strains was obtained by weighing.

### 1.2.6 Extraction of total oil by thermal-acid method

(1) A 50 ml centrifuge tube to collect the *Yarrowia lipolytica* fermentation broth and 6000 rpm high speed centrifugation for 5 min to collect the yeast bodies;
(2) 1 g of wet bacterial body was added to approximately 10 ml of 4 M hydrochloric acid, shaken well and placed in a shaker at 28°C for approximately 1-2 h;
(3) Boiling water bath for 6-8 min, immediately cool at -20°C for 30 min;
(4) Add 20 ml of chloroform: methanol = 1:1 (V/V) and mix thoroughly.
(5) Separate the lower layer of chloroform and weigh the volume, add an equal volume of 0.15% sodium chloride and centrifuge at 4000 rpm for 10 min;
(6) Transfer the lower layer to a new weighed glass tube, blow dry the solvent phase with a nitrogen blower and weigh again to calculate the oil yield, the total oil content being the ratio of the total oil yield to the dry weight.

### 1.2.7 Fatty acid methylation

(1) Add 2.6 ml of methanol: sulphuric acid = 98: 2 (V/V) solution to the glass tube, mix well and leave to react at 85°C for 3 h, after which it is cooled in the refrigerator;
(2) Add 1 ml of saturated NaCl solution and 1 ml of n-hexane, shake and centrifuge at 5000 rpm for 5 min, aspirate the supernatant into an EP tube and filter through an organic phase filter membrane before performing the gas chromatography experiment.

### 1.2.8 Analysis of fatty acid composition by gas chromatography

The fatty acid composition was analysed by gas chromatography (Agilent 7890B-GC). Chromatographic detection conditions: column was HP-INOWAX (30 m × 0.32 mm × 0.5 µm); injection temperature: 250°C; detector temperature: 250°C; injection volume: 1 µl; starting column temperature was 140°C, held for 1 min, temperature rise to 180°C at 10°C/min, held for 2 min, temperature rise to 210°C at 5°C/min, held for 4 min, then temperature rise to 250°C at 5°C/min, held for 4 min. The relative content of each fatty acid fraction was obtained by area normalization.

### 1.3 Overexpression of MaoleKCS in Yarrowia lipolytica

### 1.3.1 Construction of recombinant plasmids for overexpression of the gene

The plasmid vector was constructed using the Gibson assembly method and all assembled fragments were obtained by PCR amplification, the specific method is the same as in 1.2.2. The homologous recombinant plasmid pYLEX-FAD2-CgKCS-URA was constructed using pYLEX-URA as the backbone and was obtained directly from the patent applicant's laboratory, where the upstream and downstream of the CgKCS expression cassette were partial sequences of the FAD2 gene of *Yarrowia lipolytica* (see SEQ ID No. 14 and SEQ ID No. 15) to obtain a homologous recombinant plasmid with a targeted integration site at the FAD2 locus. The plasmid pYLEX-FAD2-CgKCS-URA was used as a backbone to construct the homologous recombinant plasmid pYLEX-FAD2-MaoleKCS-URA by replacing the CgKCS expression cassette with two consecutive MaoleKCS expression cassettes using the Gibson assembly method.

### 1.3.2 Transformation of yeast, diagnostic PCR validation of recombinant strains

The uracil nutrient-deficient strain Polg-G3-MaoleKCS-ΔURA of Polg-G3-MaoleKCS was reverse screened by the 5-FOA method. The recombinant plasmid pYLEX-FAD2-MaoleKCS-URA was digested using Not I restriction endonuclease, confirmed by agarose gel electrophoresis, and transformation of *Yarrowia lipolytica* Polg-G3-MaoleKCS-ΔURA using LiAc transformation method after recovery of fragment gum for transformation, obtaining a recombinant strain introducing the MaoleKCS gene, named as Po1g-G3-3MaoleKCS. Different transformants were picked for diagnostic PCR validation, the method is the same as that in 1.2.4, the upstream part was verified by PCR with primers FAD-df (5'-CTACGGCACGATAAAGATGG-3') and ylr-dur, and the downstream part was verified by primers ylr-ddf and FAD-dr (5'-TTACAATGGGACCGTGCTTG-3'), and upstream and downstream simultaneous validation of successful different transformants for shake flask fermentation culture, and the culture method and the analysis method of various indicators after culture were the same as in 1.2..

### 2. Results and analysis

### 2.1 Screening and expression of malania oleifera 3-ketoacyl-CoA synthase gene

### 2.1.1 Bioinformatic analysis of malania oleifera 3-ketoacyl-CoA synthase gene

Twelve 3-ketoacyl-CoA synthases were searched from the malania oleifera genome, but the functions and applications of these genes were not reported. Six of the 3-ketoacyl-CoA synthases were annotated by authors in the literature as having both KCS, KCR, HCD and ECR roles in fatty acid elongation. After multiplex protein sequence alignment of the amino acid sequences of the six KCSs with MoKCS (GenBank: QDA34238.1) and CgKCS (GenBank: ACJ61778.1) in the NCBI database, a phylogenetic tree was drawn using Neighbor joining algorithm (see Figure 1). The amino acid sequences of MaoleKCS463 and MoKCS were found to be identical, and the similarity between MaoleKCS397 and MaoleKCS398 was high. As can be seen from Figure 2, KCS398, KCS461, KCS467 and KCS817, along with the reported CgKCS and MoKCS, all contain three functionally conserved structural domains of the KCS family "FGNTSSSS" "GSGFKCNSAVW" and "GMGCSA" (red box part), indicating that these proteins belong to the KCS family proteins, and KCS398, KCS461, KCS467 and KCS817 were selected for follow-up studies. The genes of these four KCS were synthesized by Wuxi Qinglan Biotechnology Co., Ltd. after codon optimization to obtain the shuttle plasmid pMv-MaoleKCS#-AMP (# represents 398, 463, 467 and 817 numerical numbers) with MaoleKCS gene and ampicillin resistance gene.

### 2.1.2 Diagnostic PCR validation of recombinant yeast strains

Different transformants of the recombinant strain were picked from the screening plate on YPD plates, where 7 of strain Po1g-G3-MaoleKCS398 were picked and 8 of strains Po1g-G3-MaoleKCS461, Po1g-G3-MaoleKCS467 and Po1g-G3-MaoleKCS817 were each picked, while the control strain Po1g-G3 was inoculated on YPD plates and genomic DNA was extracted from different transformants of the recombinant strain and the control strain after about 24 hours of growth. PCR amplification of the upstream and downstream portions of the introduced gene MaoleKCS was performed using genomic DNA as a template to verify the integrated expression of the gene. The genomic DNA of Po1g-G3 was used as a control, and the agarose gel electrophoresis of the PCR products is shown in Figure 3. Firstly, there are no upstream or downstream PCR products in the control Po1g-G3 (see Figure 3A and Figure 3C for the leftmost lane CK); then the transformants with positive PCR results (target band of 1632 bp) in the downstream part are:
Nos. 1, 3, 4, 5 and 6 of Po1g-G3-MaoleKCS398.
Nos. 1, 2, 3 and 4 of Po1g-G3-MaoleKCS461.
Nos. 2, 3, 4, 6 and 7 of Po1g-G3-MaoleKCS467
Nos. 2, 4, 6 and 8 of Po1g-G3-MaoleKCS817 (Figure 3B);

The transformants of the recombinant strains that were positive for the downstream part were then verified by integration of the upstream part, and those that were positive for the upstream part (with a target band of 1555 bp) are:
Nos. 1, 3, 4, 5 and 6 of Po1g-G3-MaoleKCS398.
Nos. 1, 2 and 4 of Polg-G3-MaoleKCS461.
Nos. 2, 3, 4, 6 and 7 of Po1g-G3-MaoleKCS467
Nos. 2, 4, 6 and 8 of Po1g-G3-MaoleKCS817;

Combining the brightness of the PCR products of the upstream and downstream parts, the final selection of Po1g-G3-MaoleKCS

398 of No. 1, Po1g-G3-MaoleKCS461 of No. 2, Po1g-G3-MaoleKCS467 of No. 3 and Polg-G3-MaoleKCS817 of No. 8 were selected for subsequent shake flask fermentation culture.

### 2.1.3 Shake flask fermentation results of recombinant strains

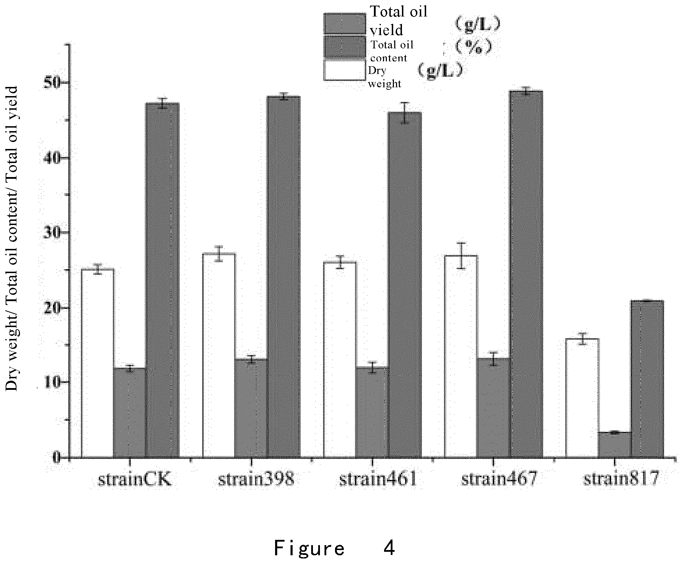

As shown in Figure 4, compared with the control strain Po1g-G3, the dry weight, total oil yield and total oil content of strain Po1g-G3-MaoleKCS817 are significantly decreased; the dry weight, total oil yield and total oil content of strains Po1g-G3-MaoleKCS398 and Po1g-G3-MaoleKCS467 are slightly increased; there is no significant change in dry weight, total oil yield and total oil content of the strains.

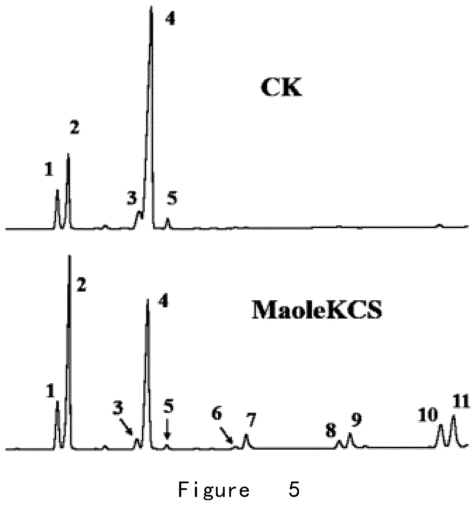

Fatty acid composition analysis shows that all strains produce significantly nervonic acid and lignoceric acid with similar yields compared to the control strain Po1g-G3, accounting for 8.9% and 6.2% of the total fatty acids, respectively (Figure 5). Meanwhile the strains have lower C18:1 and C18:0 ratios compared to the control, and a small amount of C20 and C22 fatty acids are detected (see Figure 5), indicating that MaoleKCS is able to gradually extend the carbon chains of C18:1 and C18:0 to produce nervonic acid and lignoceric acid. Therefore, the present invention has revealed that MaoleKCS, a fatty acid elongase of malania oleifera origin, can catalyze the synthesis of nervonic acid through fatty acid elongation enzyme screening and yeast in vivo expression analysis, and the corresponding gene can be used to construct high nervonic acid-producing yeast.

### 2.2 Overexpression of MaoleKCS in Yarrowia lipolytica

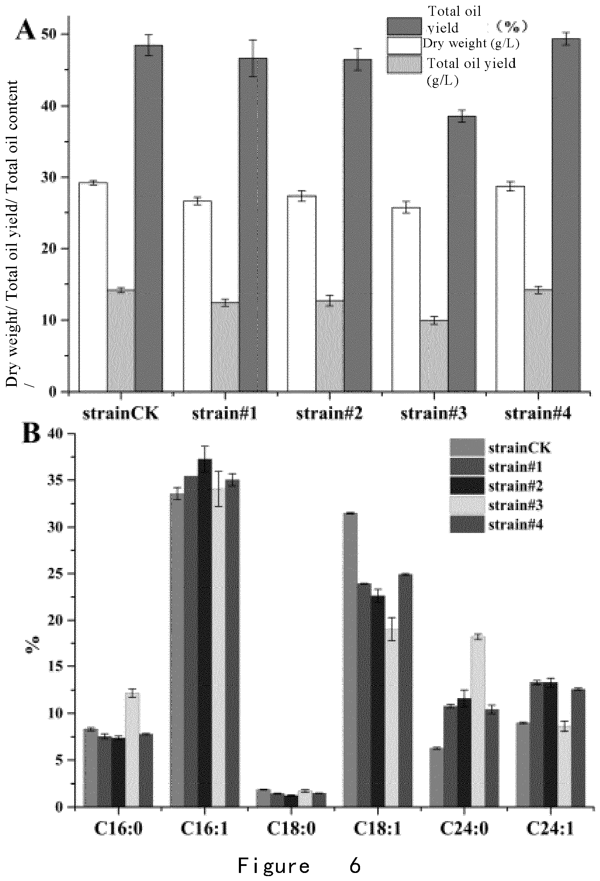

As shown in Figure 6A, compared with 29.2 g/L of the control strain, the dry weights of different transformants of the recombinant strain all decrease to different degrees, in the order of 26.7 g/L, 27.4 g/L, 25.8 g/L and 28.7 g/L. For total oil yield, the control strain is 14.16 g/L, and the transformants of No. 1, 2 and 3 decrease significantly, with No. 3 being the most significant with a decrease of 32%; The total oil yield of transformants of No.4 is 14.18 g/L, with no significant change. The total oil content is similar to the total oil yield, with significant decreases in transformants of No. 1, 2 and 3, with 3 being the most significant, and no significant change in oil content in transformant of No.4.

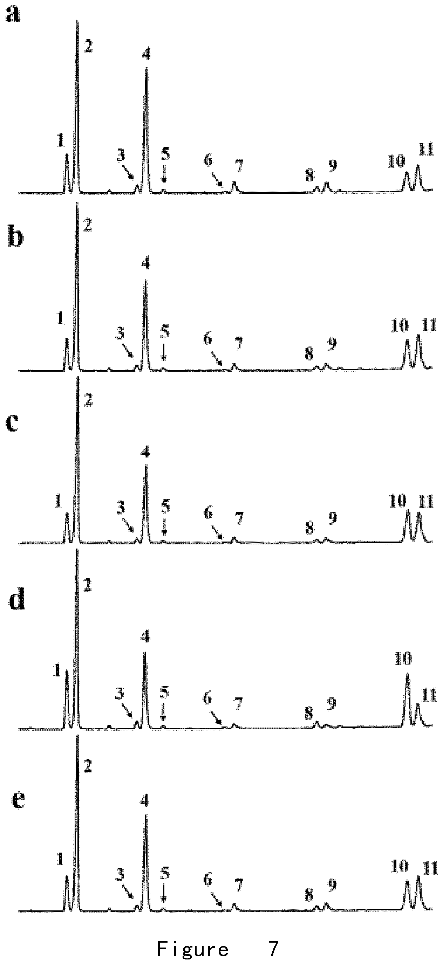

The different fatty acid ratios of each transformant of the recombinant strain are shown in Figure 6B and Figure 7. The percentage of nervonic acid to total fatty acid acid of the control strain is 8.96% (Fig. 7a), and the percentage of nervonic acid of transformants No. 1, 2 and 4 are all significantly increased, 13.27%, 13.26% and 12.60%, respectively (Fig. 7b,c,e); however, the percentage of transformant No. 3 is slightly decreased, 8.61% (Fig. 7d). Meanwhile, the proportion of lignoceric acid to total fatty acids is substantially increased in all four transformants, with transformant No.3 being the most prominent, with a nearly 2-fold increase compared to the control. Correspondingly, the proportions of both C18:0 and C18:1 in the control strain decrease to different degrees, indicating that overexpression of MaoleKCS further catalyzes carbon chain lengthening of C18:0 and C18:1, with the most significant effect of nervonic acid synthesis in transformant No. 4. The total oil yield of transformant No. 4 does not change significantly compared with the control, and the percentage of nervonic acid is increased by 40%, enhancing the ability of yeast to synthesize nervonic acid.

### Example 2: Fermentor fermentation of recombinant strains

### 1. Materials and methods

### 1.1 Fermentation medium

Seed medium (YNB medium): YNB 3 g/L, glucose 60 g/L, ammonium sulfate 35 g/L, YNB was sterilized with ammonium sulfate and added by filtration and de-bacterization.

Fermentation medium (FM medium): glucose 50 g/L, ammonium sulfate 35 g/L, yeast powder 38 g/L, potassium dihydrogen phosphate 8 g/L, sodium phosphate dibasic dodecahydrate 6 g/L, magnesium sulfate heptahydrate 5 g/L, defoaming agent 1 mL/L.

Replenishment control: controlled according to the concentration of residual sugar in the fermenter, using 600 g/L of glucose after sterilization for replenishment.

### 1.2 Fermentation process of 50 L fermenter

The conserved strain is removed from -80°C and thawed on ice. After inoculating 100 µl into 250 mL of YNB medium and incubating in shaker at 220 rpm and 28°C for 24 h, transferred to a 5 L seed fermenter at an OD of approximately 0.01 after inoculation, and the medium of the seed tank is FM fermentation medium with a filling volume of 2.5 L. The incubation conditions of the seed tank were 28°C using chillers to circulate cooling; the pH was controlled to 5.5 using 5 mol/L sodium hydroxide, DO was set at 20%, and the seeds were transferred to a 50 L fermenter (Zhenjiang Huinengda Company, Model HND-BJ-5L-50L) after 24 h of incubation to start fermentation.

FM fermentation medium was prepared according to the experimental protocol, mixed thoroughly and added into the 50 L fermenter. The fermentation tank is filled with 25 L of liquid. The inoculation rate was 10%, and 150 ml of seed solution was inserted through the inoculation port after the temperature was reduced to 28°C.

Fermentation conditions: the fermentation temperature was 28°C using chillers to circulate cooling; pH was controlled at 5.5 using 5 mol/L sodium hydroxide; the initial aeration was 1 vvm and gradually increased to a maximum aeration of 2 vvm during the first 24 h. Dissolved oxygen control method adopts and speed cascade control, with DO set at 20% and adjusted to 10% after 48 h. The rotational speed ranged from 200 rpm to 600 rpm. Supplementary glucose concentration of 600 g/L and was replenished according to the change of residual sugar concentration in the tank, and then replenished when the concentration was reduced to less than 30 g/L.

The method of analysis of indicators during fermentation was referred to Example 1. crude nervonic acid yield (g/L) = total oil yield (g/L) * ratio of nervonic acid to total fatty acid (%).

### 2. Results and analysis

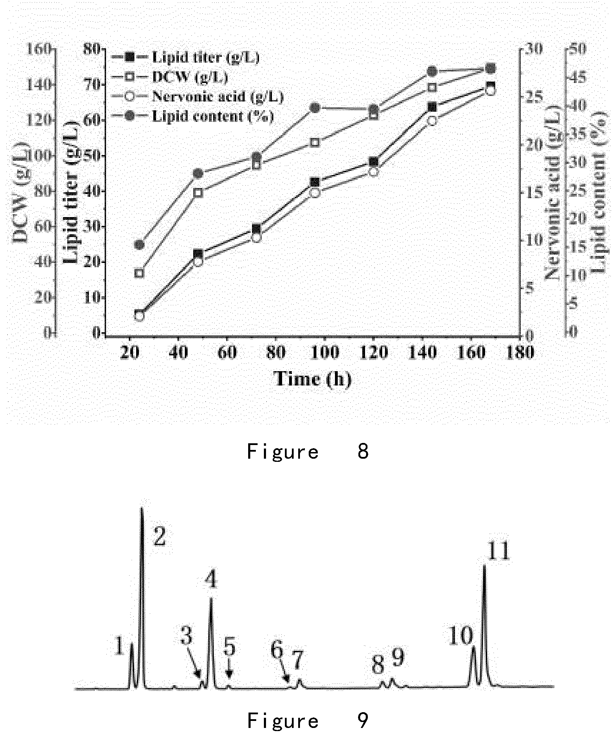

The fermentation culture of recombinant strain Polg-G3-3Maole398 was carried out on the basis of shake flask fermentation using a 50 L fermenter, and the results are shown in Figure 8. By testing the oil, dry weight and the composition of fatty acid of recombinant strain, the following conclusions are obtained: after 168 h of fermentation, the dry weight (DCW) has reached 149.4 g/L, the total oil yield (Lipid titer) is 69.6 g/L, the total oil content (Lipid Content) has reached 46.6%, the proportion of nervonic acid to total fatty acid is 36.9%, the results of gas phase analysis spectrum of fatty acid composition are shown in Figure 9, and the yield of nervonic acid has reached 25.7 g/L.

### Example 3 Mining and Analysis of Esterases in malania oleifera

Based on bioinformatics analysis, two Diacylglycerol acyltransferase (DGAT2) and three Glycerol-3-phosphate acyltransferase (GPAT) enzyme genes were obtained from the genomic data of malania oleifera (Xu, et al., 8, 2019, 8: 1-14). two DGAT2 species, named: MoDGAT2 (Maole_ 010035), MoDGAT2 (Maole_015949); three types of GPAT, named: MoGPAT (Maole_006088), MoGPAT (Maole_006089), MoGPAT (Maole_006090). 
>SEQ ID No.38
MoDGAT2(Maole_010035) Gene sequence
ATGGACGGCTCTTCGCAAATCAAGGAGCTTTCATGTCTTGTGAAATT
TGTAGAAGAAACTGTGAGGATAGAGCATGCTTCAAACCCAAATAAGCCA
GTTTATTTGGTAGGGGATTCTTTTGGTGGATGCTTGGCACTTGCTGTTGCT
GCTCGTAATCCTACATTTGATCTGGTTCTGATTTTAGTCAATCCAGCAACG
TCATTTGGCAGGTCACAGCTGCAACCTCTGCTGCCTGTCTTAGAGGCTTTG
CCGGATGGGCTTCATTTCACTGTTCCTTATCTCCTTAGCTTTATCATGGGT
ACGTGTTCTGCTGCTTTGAAGCCTTACCGTGATCCACTGAAGTTGGCAAT
GGTCAATATTGAAACTAGGTTTCCTCCTGCACTAGTACTAGAGCAATTGT
CTGGCAACCTCACAGCTTTGCTACCACGCCTTTCTGGCTTGGCTAATATTA
TACCGAAGGAGACTCTTCTTTGGAAGCTAAAGCTCCTAAAATCAGCTGCT
GCTTATGCTAATTCACGTCTCCATGCTGTTAAAGCTGAAGTACTTGTTCTG
GCTAGTGGCAGTGATAACATGCTACCTAGCAAAGATGAAGCTGAACGCCT
TTCACGTTCATTACAGAATTGCATAGTTCGTCTCTTTAAGGACAATGGGC
ATACCCTTCTATTGGAAGATGGCTATAGTCTGCTGACCATCATTAAATGT
ACTTCCAAATACCGTTGTTCAAGGAAGCATGATTTCATCACGGATTTTCTG
CCTCCAAGTATGTCTGAATTCAAACAGGAATTGAACCAGAGATTTGGGTT
ATTTCGTGTTGCTACTAGTCCCATACTGTTCTCAACACTACCAGATGGGAA
GATAGTTAAAGGTCTTGCCGGAATCCCAAATGAAGGTCCCGTCTTATTAG
TTGGTTACCACATGTTGTTGGGATGTGAACTAGGTCCACTTGTTGAAGCA
TTCTTGAAAGAGAAGAATATTATGATTCGCGGTGTAGCGCACCCAGAGAT
GTTTTCAAAGAAGCATGAAGGTCCATCAAATGAATTTGGACCTTTTGATT
TGGTGAAACTGTTTGGTGGCCTTCCTGTCACACCAAGAAACCTTTTCAAA
TTGTTTTCAACAAAATCACATATACTCCTTTATCCAGGAGGTGCACGTGA
GGCTCTTCATCGCAAGGGCGAAAAGTACAAGTTATTGTGGCCCAACCAGC
CTGAATTTGTTAGAATGGCTGCAAGATTTGGTGCCACCATTGTACCGTTTG
GGACTGTTGGAGAAGACGACATAGCAGAAGCGGAATCGACTATGTTGGC
AAAGAAGATCACGCTGAGGAGCTCTGACGGCGAGACCTTTGAGGTTGAC
AAGATCGTGGCTCTTGAGTCTCAGATGATCAAGCACATGATTGAGGATGA
CTGTGCGGACAATGGAATACCCTTGCCCAACGTCACCAGCTGGATCTTGG
TGAAGGTCATCGAGTACTGCAAGAAGCACGTCAAGGCTCCCAAGATCGA
GAAACGAGGCGACGTCAACAAGGAGCTCAAGTCGTGGGACGCTGAGTTT
 GTCAAAATTGACTAG
(a) Sequence characteristics:
   - Length: 1566
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Diacylglycerol acyltransferase (DGAT). 
>SEQ ID No.39
MoDGAT2(Maole_010035) amino acid sequence
MDGSSQIKELSCLVKFVEETVRIEHASNPNKPVYLVGDSFGGCLALAVA
ARNPTFDLVLILVNPATSFGRSQLQPLLPVLEALPDGLHFTVPYLLSFIMGTCS
AALKPYRDPLKLAMVNIETRFPPALVLEQLSGNLTALLPRLSGLANIIPKETLL
WKLKLLKSAAAYANSRLHAVKAEVLVLASGSDNMLPSKDEAERLSRSLQNC
IVRLFKDNGHTLLLEDGYSLLTIIKCTSKYRCSRKHDFITDFLPPSMSEFKQEL
NQRFGLFRVATSPILFSTLPDGKIVKGLAGIPNEGPVLLVGYHMLLGCELGPL
VEAFLKEKNIMIRGVAHPEMFSKKHEGPSNEFGPFDLVKLFGGLPVTPRNLF
KLFSTKSHILLYPGGAREALHRKGEKYKLLWPNQPEFVRMAARFGATIVPFG
TVGEDDIAEAESTMLAKKITLRSSDGETFEVDKIVALESQMIKHMIEDDCAD
NGIPLPNVTSWILVKVIEYCKKHVKAPKIEKRGDVNKELKSWDAEFVKID
(a) Sequence characteristics:
   - Length: 521
   - Type: amino acid sequence
      - Chain type: single-stranded
      - Topology: Linear
(b) Molecular type: amino acid
(c) Hypothesis: No
(d) Antisense: No
(e) Original source: malania oleifera

Sequence features: the amino acid sequence is the amino acid sequence of Diacylglycerol acyltransferase (DGAT). 
>SEQ ID No.40
MoDGAT2(Maole_015949) Gene sequence
ATGGCGGAGACCGAGGTCAGAGAGTCGCCGTTGACCGCCGCCCCTG
CCCCCGAGGTGACTGTCATCGAGAGCCCCCGAGTTCCCCTGCTCCATTCC
GTGCTCGCCACCGCCCTGTGGATTGGTTGCATCCACCTGTTCTTCGCCGTC
ATGTTCACCGCCACTTTCCTGCTCCCCCTCTCCAAATCCATTGCGGTGTTT
GCTCTGCTACTGGTTCTTATTGTGGTTCCTGTTGAGGCTGATAGTAAATTT
GGGATGAAAGTAAGGAGGGGCAATAACCATCATGATAATGAAGGTGATG
ATGAAGAGTTCTCCAACATACATGGTTGGGTTGTAAAAAAAATGGTGGTG
GTTTATTGCCACAGTGTCACTATGCCAGAACTGGAGGTGACAATGCTGCC
 
AAGTAACGGTTCTGTTTTGTCCTTCACAGTCTTTGCGTTGGAGCCACATTC
AGTGTTGCCTGTTGGTGTCATCTCACTTATGCACCTTTCAAATGCTGTGCC
CCTCCCAAAAACTAGGGTTCTTGCCAGTAGCGCTGTGTTTAGAACACCAT
TCCTGAGGCATATATGGACATGGATGGGCCTTGCAGCTGTAACAAGGAAA
AATTTTATCTCCCTTTTGGCAGCTGGTTATAGTTGCGCCATAATACCAGGT
GGGACGAGGGAGACATTGCTTATGGTGCAAGATCATGAGGTTTATCTCTT
GAAACTCTCTGTGGCTCTATTGCGTATTGCTGCACGCTCTACAGCTTTGGA
GTGGAACTTGTGCCCATTCCAGAAGATTGCTTTCCTTAAGACGAGAAAAG
GATTTGTTCGCACGGCTATAGAGACCGGTGTGCCTCTAATCCCTGTTTTCT
CTTTCGGCCAGGTATTTTTTTTTTTTTGGAAAGATCAAGTTTACACCGATG
TTCTTCTGGGGAGTTTTGGGATGCTACATCTCATAACATGGACAGGGCTC
CTTTCCACACATCACACACGAATGCCTTCTTTCTTCATCATGAGGAGAGCT
GCCACTGTTCCCCTAACTCCTATGCCTCGTCGGCTTCCATTGCATGTGGTT
GTGGGTAGACCAATTGAGGTTAAGCAAAATTCACAACCCACAGCAGAAG
AGGTGAATGAAGTGCACAGTCAGTTTGTGGGGGCATTGCAAGATCTGTTC
GAGAGGCACAAAGCAAGGGTTGGGCACGCTGATCGTGAGCTGAAAATCA
TTTGA
(a) Sequence characteristics:
   - Length: 1209
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Diacylglycerol acyltransferase (DGAT). 
>SEQ ID No.41
MoDGAT2(Maole_015949) amino acid sequence
MAETEVRESPLTAAPAPEVTVIESPRVPLLHSVLATALWIGCIHLFFAVM
FTATFLLPLSKSIAVFALLLVLIVVPVEADSKFGMKVRRGNNHHDNEGDDEE
FSNIHGWVVKKMVVVYCHSVTMPELEVTMLPSNGSVLSFTVFALEPHSVLP
VGVISLMHLSNAVPLPKTRVLASSAVFRTPFLRHIWTWMGLAAVTRKNFISL
LAAGYSCAIIPGGTRETLLMVQDHEVYLLKLSVALLRIAARSTALEWNLCPF
QKIAFLKTRKGFVRTAIETGVPLIPVFSFGQVFFFFWKDQVYTDVLLGSFGML
HLITWTGLLSTHHTRMPSFFIMRRAATVPLTPMPRRLPLHVVVGRPIEVKQNS
QPTAEEVNEVHSQFVGALQDLFERHKARVGHADRELKII
(a) Sequence characteristics:
   - Length: 402
   - Type: Amino acid sequence
      - Chain type: Single-stranded
      - Topology: Linear
(b) Molecular type: amino acid
(c) Hypothesis: No
(d) Antisense: No
(e) Original source: malania oleifera

Sequence features: The amino acid sequence is the amino acid sequence of Diacylglycerol acyltransferase (DGAT). 
>SEQ ID No.42
MoGPAT(Maole_006088) Gene sequence
ATGGACTTTGTACCCGACATCTCCGCCATCAAGACCGACAAGATTAA
AATTGACAAGGAAACCACCGACATGCTTACCGCTCGGCATGACGGATCTT
CTGAAGTTAGGGAGGACGAGTTGGTGGCTGCGCCAGTGCCAACTTCTCCG
TTCGGTTGTTGGGCCGGCGGACCCGGTAGGAGCATCAACCGTCGCACAAA
TCAAATACCTCTCGAACCTTGCAGAGACATCTCAGTTCATAAACTAATCG
AACCGATGTGCTCAAAAAGACTAGGGAGTGATTTCTCCGACCAGTTAGCG
TCACCTTGTGTGTCTCATTCGTGGTTGCCGGCGACGACGAAGTGCGATCA
TGTGCACTGCCTGTGCATGGAACGTGCCTGCTGCTGGAATTGGTCCTCTG
ATGATGTGCTTTCAGCTGCTGGCGGATCAAGTCGTGTTGAATCTAATAGT
GTTCATGGCGTATTAAGAAAACTCCAGCAATGGTCTGCAATGAACAGTGC
TGCCGATGGGGGGACAAAGGGGCTGGAATTTGACAGAAAAGACACGAAG
AACAAGAATGTGATGAAAGAGAGTACAGTGACCACTCCAAAGACCTTGA
GGGTTGCATTGAAATGCTGCACAAGGGAAGATACTGCACCGGAGGCAAA
AGAGAATATGAATTATTTCCGTGGTATAGAATGTTGTGAGGTTAAGGAGG
CATATTCGAAGGCACTGCATGATTCTGTTACTGAACAATACAATGTGCTC
AAATCTGCCATACATGGCAAACAAGGACTGGAGGCATCCATTCCAGATGT
CTCATTATCACAACCATGGCAGTAG
(a) Sequence characteristics:
   - Length: 819
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Glycerol-3-phosphate acyltransferase (GPAT). 
>SEQ ID No.43
MoGPAT(Maole_006088)    Amino acid sequence
MAETEVRESPLTAAPAPEVTVIESPRVPLLHSVLATALWIGCIHLFFAVM
FTATFLLPLSKSIAVFALLLVLIVVPVEADSKFGMKVRRGNNHHDNEGDDEE
FSNIHGWVVKKMVVVYCHSVTMPELEVTMLPSNGSVLSFTVFALEPHSVLP
VGVISLMHLSNAVPLPKTRVLASSAVFRTPFLRHIWTWMGLAAVTRKNFISL
LAAGYSCAIIPGGTRETLLMVQDHEVYLLKLSVALLRIAARSTALEWNLCPF
QKIAFLKTRKGFVRTAIETGVPLIPVFSFGQVFFFFWKDQVYTDVLLGSFGML
HLITWTGLLSTHHTRMPSFFIMRRAATVPLTPMPRRLPLHVVVGRPIEVKQNS
QPTAEEVNEVHSQFVGALQDLFERHKARVGHADRELKII
(a) Sequence characteristics:
   - Length: 402
   - Type: Amino acid sequence
      - Chain type: Single-stranded
      - Topology: Linear
(b) Molecular type: amino acid
(c) Hypothesis: No
(d) Antisense: No
(e) Original source: malania oleifera

Sequence features: The coding product of this gene is Glycerol-3-phosphate acyltransferase (GPAT). 
>SEQ ID No.44
MoGPAT(Maole_006089) Gene sequence
ATGGTGCTGTGGCAGTGCGGTGGGTCTGGACTCAGAGGCAGCGATG
AAGGTTTGCGATGGCACGGCGCTGCTATGCTTGCAACTCGCACGGTGCCG
GCAGCGGCAGGTCTGGACAGTGGACGCCACTCGCACAATGTCCAAGGTA
GCCAACCTCAAATCCAGGAGATCTGTGCGAAAGAGAGCTTGGGGAATAT
TGACGGTGTGAGGGGTGACTCTATTAATGATGCGGAGTGGGCTGGTAAGG
CTGTGACCTATGTAGCAGGGGATCGGGTTTTAACGGATATTCTTTGCAAG
CCCTTCAGCATGGGCAGAAATCTTATGTGCGTCTACTCAAAAAAGCACAT
GCTTGATGATCCTAAGCTTGCTGAGATGAAGAAAAAAGCAAATATACGA
AGTTTGAAAGAAATGGCCATGCTTTTGAGAGGAGGGTCACAAATAGTATG
GATTGCACCAAGTGGTGGTAGGGACCGTCCAGATCCCCAGACAGGAGAA
TGGCATCCGGCACCCTTTGATGCTTCTTCAGTGGACAATATGAGGCGGCT
TGCAGAAAATGCTGGTGTTCCTGCACATATTTATCCATTAGCATTAATTTG
CTACAACATTATGCCGCCTCCACCCAAGGTTGAGAAAGAGATTGGAGAG
AAAAGAGTGGTATCCTTTCATGGGGTTGGACTATCCGTGATACCAGATAT
AAGCTATGCTGAAATTGCCGCTGCTTGTGAAAACTCTGAAGAGACTGGCG
ATGCCGACACAAATCAGTCACACTACCAATGTCATCGCAGGATCCCGCAG
GCGGAGAAATTTGGCATCTTGGCCGCATTGCCCGATGATGAGTCAAGGGC
GAAGGCCACATCTCCCAGATCTACGCCATCCAAGCGCCTTCGTTACAAGA
TCGCCGGCTTCTCCGCCCACCTCATGAAGCGAATCCAGAAGGGTCCCTTT
CGGGGTATTTCTCTGAAGCTGCAAGAGGAAGAGCACGAGAGGCGCATGG
ACTTCGTACCCGACATCTCCGCCATCAAGACCGACAAGATTAAAGTCGAC
AAGGAAACCAGATCTACACCATCCAGGCGCCTTCGCAACAAGATCGCGA
CTTCTCCACCCACCTCATGA
(a) Sequence characteristics:
   - Length: 1110
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Glycerol-3-phosphate acyltransferase (GPAT). 
>SEQ ID No.45
MoGPAT(Maole_006089)    Amino acid sequence
MVLWQCGGSGLRGSDEGLRWHGAAMLATRTVPAAAGLDSGRHSHNV
QGSQPQIQEICAKESLGNIDGVRGDSINDAEWAGKAVTYVAGDRVLTDILCK
PFSMGRNLMCVYSKKHMLDDPKLAEMKKKANIRSLKEMAMLLRGGSQIVW
IAPSGGRDRPDPQTGEWHPAPFDASSVDNMRRLAENAGVPAHIYPLALICYN
IMPPPPKVEKEIGEKRVVSFHGVGLSVIPDISYAEIAAACENSEETGDADTNQS
HYQCHRRIPQAEKFGILAALPDDESRAKATSPRSTPSKRLRYKIAGFSAHLMK
RIQKGPFRGISLKLQEEEHERRMDFVPDISAIKTDKIKVDKETRSTPSRRLRNK
IATSPPTS
(a) Sequence characteristics:
   - Length: 272
   - Type: Amino acid sequence
      - Chain type: Single-stranded
      - Topology: Linear
(b) Molecular type: amino acid
(c) Hypothesis: No
(d) Antisense: No
(e) Original source: malania oleifera

Sequence features: The coding product of this gene is Glycerol-3-phosphate acyltransferase (GPAT). 
>SEQ ID No.46
MoGPAT(Maole_006090) Gene sequence
ATGCAGCTAAGAATACTTTTAGATGTTTTGACTCTTCTGTTTGCAGCA
TTTCTTTATTTGGAATCTAATTTGGAGGTATTGTTTTCTTGTCTTGCTGGGT
TTCCTGCAGAGCTGCTTTCTGGAATAAAGAAGGAAACAGAAGCTGGCAG
ATTGCCTTCAAATTTTGCTTCAGTTCTGGTTTTCCAAAGTGGAATTCCAGA
TGCTGATGAAATTATATTGTCAAACACAACTGCTTTGTTTGATCGTGTTTT
ACTGGATGCAGAGGACTCTTTTGTTTTCCCCCCACATCATAAAGCAATGC
GAGAGCCTTTTGATTACTACATGTTTGGTCAAAATTATATCCGTCCTTTGA
TAGATTTTGGGAATTCATATGTTGGCAATATCAACATTTTTCATGAAATGG
AAGAGAAGCTGCAGCAGGTCAAGAATCTCAATGGGATTGAGTGGGGTCA
GCCATGGAGGAGTCTCCACTCTCTGCAAATAGAGAGTAAATCCTTAGATC
TGCGACTGGAAAAGGTTGGGAAGGGTTTCTTTTTGGCTGGATGCTGTTTT
CGGTTGCAAAGGGAAATTGTCAAGTTAAATTCTTGGATTTGGGGTTATAC
TCATTTGGAAAGGCTTATTCTATTTTTGTTCTATGGGGTTTTGATGGGGAG
TGATGGCAACGGTTTGTTGAGATGTTGCAGCCGACGGTAA
(a) Sequence characteristics:
   - Length: 693
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is g Glycerol-3-phosphate acyltransferase (GPAT). 
>SEQ ID No.47
MoGPAT(Maole_006090) amino acid sequence
MQLRILLDVLTLLFAAFLYLESNLEVLFSCLAGFPAELLSGIKKETEAGR
LPSNFASVLVFQSGIPDADEIILSNTTALFDRVLLDAEDSFVFPPHHKAMREPF
DYYMFGQNYIRPLIDFGNSYVGNINIFHEMEEKLQQVKNLNGIEWGQPWRSL
HSLQIESKSLDLRLEKVGKGFFLAGCCFRLQREIVKLNSWIWGYTHLERLILF
LFYGVLMGSDGNGLLRCCSRR
(a) Sequence characteristics:
   - Length: 230
   - Type: Amino acid sequence
      - Chain type: Single-stranded
      - Topology: Linear
(b) Molecular type: amino acid
(c) Hypothesis: No
(d) Antisense: No
(e) Original source: malania oleifera

Sequence features: The coding product of this gene is Glycerol-3-phosphate acyltransferase (GPAT).

The codons of the above five esterases are optimized according to Yarrowia lipolytica and the sequences are shown below: 
>SEQ ID No.48
MoDGAT2(Maole_010035) Gene sequence
ATGGACGGCTCTTCTCAGATCAAGGAGCTGTCTTGCCTGGTGAAGTT
CGTGGAGGAGACTGTGCGAATCGAGCACGCCTCTAATCCCAATAAGCCCG
TGTACCTGGTGGGCGACTCTTTCGGCGGATGTCTGGCTCTGGCTGTGGCT
GCTAGAAATCCTACTTTCGACCTGGTGCTGATCCTGGTGAACCCCGCCAC
TTCTTTCGGCAGATCTCAGCTGCAACCTCTGCTGCCCGTGCTGGAGGCTCT
GCCTGATGGACTGCATTTCACCGTGCCCTACCTGCTGTCTTTCATCATGGG
CACCTGCTCTGCCGCCCTGAAACCCTATAGAGATCCCCTGAAACTGGCCA
TGGTGAACATCGAGACTCGATTCCCCCCCGCCCTGGTGCTGGAACAACTT
TCTGGAAATCTGACCGCCCTGCTGCCCAGACTGTCTGGACTGGCTAATAT
CATCCCCAAGGAAACCCTGCTGTGGAAGCTGAAGCTGCTGAAGTCTGCCG
CCGCCTATGCTAATTCTCGACTGCACGCCGTGAAAGCCGAGGTGCTGGTG
CTGGCTTCTGGATCTGATAACATGCTGCCCTCTAAGGACGAGGCCGAGCG
ACTTTCTAGATCTCTGCAAAATTGCATCGTGCGACTGTTCAAGGACAACG
GCCACACCCTGCTGCTGGAGGATGGATATTCTCTGCTGACCATCATCAAG
TGCACCTCTAAGTACCGATGCTCTCGAAAGCACGACTTCATCACCGACTT
CCTGCCCCCCTCTATGTCTGAGTTCAAGCAGGAGCTGAACCAGCGATTCG
GCCTGTTCCGAGTGGCCACTTCTCCTATTCTGTTTTCTACCCTGCCCGACG
GCAAAATCGTGAAGGGCCTGGCTGGAATTCCCAATGAGGGCCCTGTGCTG
CTGGTGGGCTATCATATGCTGCTGGGATGTGAGCTGGGCCCCCTGGTTGA
AGCTTTTCTGAAAGAGAAAAACATCATGATCCGAGGCGTGGCCCACCCCG
AAATGTTTTCTAAAAAGCACGAGGGCCCCTCTAACGAGTTCGGCCCTTTC
 
GATCTGGTGAAGCTGTTCGGCGGACTGCCCGTGACTCCTAGAAATCTGTT
TAAGCTGTTCTCTACCAAGTCCCACATCCTGCTGTACCCCGGCGGCGCTA
GAGAAGCTTTACATAGAAAAGGCGAAAAGTACAAGCTGCTGTGGCCCAA
CCAGCCCGAGTTTGTGAGAATGGCTGCTCGATTTGGCGCCACTATCGTGC
CTTTCGGAACCGTGGGAGAGGACGACATTGCCGAGGCTGAGTCTACCATG
CTGGCTAAGAAAATCACCCTGCGATCTTCTGACGGCGAAACCTTCGAGGT
GGACAAGATCGTGGCCCTGGAGTCTCAGATGATCAAGCACATGATCGAG
GACGACTGCGCCGACAACGGCATTCCTTTACCTAATGTGACCTCTTGGAT
CCTGGTGAAGGTGATCGAGTACTGCAAGAAGCACGTGAAGGCCCCCAAG
ATCGAGAAGCGAGGCGATGTGAATAAGGAGCTGAAGTCTTGGGACGCCG
AGTTCGTGAAGATCGACTAA
(a) Sequence characteristics:
   - Length: 1566
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Diacylglycerol acyltransferase (DGAT). 
>SEQ ID No.49
MoDGAT2(Maole_015949) Gene sequence
ATGGCCGAGACTGAAGTGCGAGAGTCTCCCCTGACTGCCGCCCCTGC
TCCTGAGGTGACTGTGATTGAATCTCCCAGAGTGCCCCTGCTGCACTCTGT
GCTTGCTACTGCTCTGTGGATCGGCTGTATTCATCTGTTTTCGCCGTGAT
GTTCACCGCCACCTTCCTGCTGCCTCTGTCTAAATCTATCGCCGTGTTCGC
CCTGCTGCTGGTGCTGATTGTGGTGCCCGTTGAAGCCGATTCTAAGTTCG
GCATGAAGGTGCGACGAGGCAACAACCACCACGACAACGAGGGCGATGA
CGAGGAGTTTTCTAACATCCACGGCTGGGTGGTGAAGAAGATGGTGGTGG
TGTACTGCCACTCTGTGACCATGCCCGAGCTGGAGGTTACTATGCTGCCC
TCTAATGGCTCTGTGCTGTCTTTCACCGTGTTCGCCCTGGAGCCCCACTCT
GTGCTGCCTGTTGGAGTTATTTCTCTGATGCACCTGTCTAACGCCGTGCCC
CTGCCTAAAACTAGAGTGCTGGCTTCTTCTGCCGTGTTCCGAACCCCTTTC
CTGCGACATATCTGGACCTGGATGGGCCTGGCCGCTGTTACTAGAAAAAA
TTTCATCTCTCTGCTGGCCGCCGGCTACTCTTGCGCTATTATTCCCGGAGG
AACTCGAGAAACCCTGCTGATGGTGCAAGACCACGAGGTGTACCTGCTGA
AGCTGTCTGTGGCCCTGCTGCGAATTGCCGCTAGATCTACTGCCCTGGAA
TGGAACCTGTGTCCCTTTCAGAAAATCGCCTTCCTGAAGACCCGAAAGGG
CTTCGTGCGAACCGCCATTGAAACCGGAGTGCCCCTTATTCCTGTGTTCTC
TTTCGGCCAGGTGTTCTTCTTCTTCTGGAAGGACCAGGTGTACACCGACGT
GCTGCTGGGCTCTTTTGGCATGCTGCACCTGATCACCTGGACCGGCTTACT
GTCTACTCACCATACCCGAATGCCCTCTTTCTTCATCATGCGACGAGCCGC
CACCGTGCCCCTTACTCCCATGCCTAGAAGACTGCCTCTGCATGTGGTGG
TGGGACGACCTATTGAGGTGAAACAGAACTCTCAGCCCACCGCCGAGGA
AGTGAATGAGGTGCATTCTCAGTTCGTGGGCGCCCTGCAAGATCTGTTCG
 
AGCGACATAAGGCCAGAGTGGGCCACGCCGATAGAGAACTGAAAATCAT
TTAA
(a) Sequence characteristics:
   - Length: 1209
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: the coding product of this gene is Diacylglycerol acyltransferase (DGAT). 
>SEQ ID No.50
MoGPAT(Maole_006088) Gene sequence
ATGGACTTCGTGCCCGACATCTCTGCCATCAAGACCGACAAGATCAA
GATCGACAAGGAGACTACCGACATGCTGACCGCCAGACATGATGGCTCTT
CTGAGGTGAGAGAAGACGAGCTGGTGGCCGCCCCTGTGCCTACTTCTCCT
TTTGGATGTTGGGCTGGCGGACCCGGCAGATCTATCAACAGAAGAACTAA
TCAGATCCCCCTGGAGCCCTGCCGAGACATTTCTGTGCATAAACTGATCG
AGCCCATGTGCTCTAAGCGACTGGGCTCTGACTTCTCTGACCAGCTGGCC
TCTCCCTGCGTGTCTCACTCTTGGCTGCCTGCTACTACCAAATGCGACCAT
GTGCACTGCCTGTGCATGGAGCGAGCCTGTTGTTGGAATTGGTCTTCTGA
CGACGTGCTGTCTGCCGCCGGAGGATCTTCTAGAGTGGAATCTAACTCTG
TGCACGGCGTGCTGCGAAAGCTGCAGCAATGGTCTGCCATGAACTCTGCC
GCCGATGGAGGCACTAAAGGACTGGAGTTTGATCGAAAGGACACCAAGA
ACAAGAACGTGATGAAGGAGTCTACCGTGACCACCCCCAAGACCCTGAG
AGTGGCTCTGAAATGCTGTACCCGAGAGGATACCGCCCCCGAAGCTAAA
GAGAACATGAACTACTTCCGAGGCATCGAGTGCTGCGAGGTGAAGGAGG
CTTATTCTAAGGCCCTGCACGACTCTGTGACCGAGCAGTATAATGTGCTG
AAGTCTGCCATCCACGGCAAGCAGGGCCTGGAGGCTTCTATTCCCGATGT
GTCTCTGTCTCAGCCCTGGCAGTAA
(a) Sequence characteristics:
   - Length: 819
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Glycerol-3-phosphate acyltransferase (GPAT). 
>SEQ ID No.51
MoGPAT(Maole_006089) Gene sequence
ATGGTGCTGTGGCAGTGCGGAGGATCTGGACTGAGAGGATCTGACG
AAGGACTGCGATGGCACGGCGCTGCTATGTTAGCTACTAGAACTGTGCCT
 
GCTGCTGCTGGACTGGACTCTGGAAGACATTCTCATAACGTGCAGGGCTC
TCAGCCCCAGATCCAAGAGATTTGCGCCAAGGAGTCTCTGGGCAACATCG
ACGGCGTGCGAGGAGATTCTATTAACGACGCCGAATGGGCCGGCAAAGC
CGTGACTTATGTGGCTGGCGATCGAGTGCTGACCGATATTCTGTGCAAGC
CCTTCTCTATGGGCCGAAACCTGATGTGCGTGTACTCTAAGAAGCACATG
CTGGACGACCCCAAGCTGGCCGAAATGAAGAAGGCCAACATCCGAT
CTCTGAAGGAGATGGCCATGCTGCTGCGAGGCGGATCTCAAATCGTGTGG
ATCGCCCCCTCTGGCGGCAGAGATAGACCTGATCCTCAAACTGGAGAGTG
GCACCCCGCTCCCTTTGACGCTTCTTCTGTGGACAACATGCGACGACTGG
CCGAGAACGCCGGAGTTCCTGCTCATATTTATCCTCTGGCTCTGATCTGTT
ACAACATCATGCCCCCTCCCCCCAAGGTGGAAAAAGAGATTGGAGAGAA
GCGAGTGGTGTCTTTCCACGGCGTGGGCCTTTCTGTGATTCCCGACATTTC
TTACGCCGAGATCGCCGCCGCTTGTGAAAACTCTGAAGAGACTGGAGATG
CCGACACCAACCAGTCTCATTATCAGTGCCACCGACGAATCCCCCAGGCT
GAAAAATTTGGCATTCTGGCCGCTCTGCCTGACGATGAGTCTCGAGCTAA
AGCCACCTCTCCCCGATCTACCCCTTCTAAACGACTGCGATACAAGATCG
CCGGCTTCTCTGCCCACCTGATGAAGCGAATTCAGAAGGGCCCCTTCCGA
GGCATTTCTCTGAAGCTGCAGGAGGAGGAGCACGAGCGACGAATGGATT
TCGTGCCCGACATTTCTGCCATCAAGACCGACAAGATCAAGGTGGACAAG
GAAACCCGATCTACCCCCTCTCGACGACTGCGAAATAAGATCGCCACCTC
TCCCCCCACCTCTTAA
(a) Sequence characteristics:
   - Length: 1110
   - Type: DNA sequence
      - Strand type: single-stranded
      - Topology: Linear
(b) Molecular type: DNA
(c) Hypothesis: No
(d) Antisense: No
(e) Original source: malania oleifera

Sequence features: The coding product of this gene is Glycerol-3-phosphate acyltransferase (GPAT). 
>SEQ ID No.52
MoGPAT(Maole_006089) Gene sequence
ATGCAGCTGCGAATCCTGCTGGACGTGCTGACCTTACTGTTTGCCGC
TTTTCTGTACCTGGAGTCTAACCTGGAGGTGCTGTTCTCTTGCCTGGCCGG
CTTTCCTGCTGAACTGCTGTCTGGAATTAAGAAGGAGACTGAGGCCGGCC
GACTGCCCTCTAATTTTGCATCTGTGCTGGTGTTCCAGTCTGGCATCCCCG
ACGCTGATGAAATCATTCTGTCTAACACCACCGCCCTGTTCGACCGAGTG
CTGCTTGATGCTGAAGACTCTTTCGTGTTCCCCCCCCACCACAAGGCCATG
AGAGAACCTTTTGATTACTACATGTTCGGCCAGAACTACATCCGACCCCT
GATCGACTTCGGCAACTCTTACGTGGGCAACATCAACATCTTCCACGAGA
TGGAGGAGAAGCTGCAGCAGGTGAAGAACCTGAACGGCATCGAGTGGGG
CCAGCCCTGGAGATCTCTGCATTCTCTGCAGATCGAGTCTAAGTCTCTGG
ACCTGCGACTGGAGAAGGTGGGCAAAGGATTTTTCCTGGCCGGATGCTGT
TTTCGACTGCAGCGAGAGATCGTGAAGCTGAACTCTTGGATCTGGGGCTA
CACCCACCTGGAGCGACTGATCCTTTTTCTGTTCTACGGCGTGCTGATGGG
 CTCTGACGGCAATGGACTGCTGAGATGTTGCTCTCGACGATAA
(A) SEQUENCE CHARACTERISTICS:
   - LENGTH: 693
   - TYPE: DNA SEQUENCE
      •STRAND TYPE: SINGLE-STRANDED
      • TOPOLOGY: LINEAR
(B) MOLECULAR TYPE: DNA
(C) HYPOTHESIS: NO
(D) ANTISENSE: NO
(E) ORIGIN: malania oleifera

SEQUENCE FEATURES: THE CODING PRODUCT OF THIS GENE IS Glycerol-3-phosphate acyltransferase (GPAT).

### Example 3 Synthesis, plasmid construction and yeast transformation of esterases in malania oleifera

The genes MoDGAT2 (Maole_010035), MoDGAT2 (Maole_015949), GPAT, MoGPAT (Maole_006088), MoGPAT (Maole_006089) and MoGPAT (Maole_006090) were synthesized by codon optimization at Wuxi Qinglan Biotechnology Co., LTD (the sequences of the optimized genes are SEQ ID No. 48, 49, 50, 51 and 52 in order) to obtain shuttle plasmids with esterase gene and ampicillin resistance gene, named as pMV-MoDGAT2(Maole_010035)-AMP, pMV- MoDGAT2(Maole_015949)-AMP, and pMV-MoGPAT(Maole_006088)-AMP, pMV-MoGPAT(Maole_006089)-AMP, pMV- MoGPAT(Maole_006090)-AMP.

The plasmid vectors were constructed using the Gibson assembly method and all assembled fragments were obtained by PCR amplification. The plasmid backbone was derived from the pYL-PEX10-CgKCS plasmid (obtained from the Qingdao Institute of Bioenergy and Processes, Chinese Academy of Sciences). The esterase promoter used is TEFintro. All PCR amplifications were carried out using KAPA HiFi high fidelity DNA polymerase in a 50 µl amplification system (2× KAPA Mix, 25 ul; 1.5 ul of each 10 µM primer; 1 µl of template; fill to 50 µl with water) to obtain each DNA sequence. Amplification conditions were: pre-denaturation at 95°C for 3 min; denaturation at 98°C for 20 sec, annealing at 60-72°C for 15 sec, extension at 72°C, extension time based on 30 seconds per kb, number of cycles 29-35; extension at 72°C for 10 minutes.

After Gibson assembly of the backbone and esterase gene fragment and transformation of E. coli competent Trans-T1 (Beijing All-style Gold Biotechnology Co., LTD), the homologous recombinant plasmid pYLEX-PEX10-MoDGAT2 (Maole_010035)-URA, pYLEX-PEX10-MoGPAT (Maole_006088)-URA, pYLEX-PEX10-MoGPAT (Maole_006089)-URA, pYLEX-PEX10- MoGPAT (Maole_006090)-URA was obtained by colony PCR with primers TEFin-PF and XPR2-PR and validated by sequencing (Tsingke Biotechnology Co., Ltd.). The primers are shown in Table 1.

**Table 1. Primers used for plasmid construction and colony PCR**

| Primer name | Primer sequences | Template name |
|---|---|---|
| PEX10-35-f | gatcgactaaccccacgttgccggtctt | PYL-Maole016461_PEX10 |
| PEX10-35-r | agccgtccatctgcggttagtactgcaaaaagtgc | |
| PEX10-35-F | ctaaccgcagatggacggctcttctcag | pMV-MoDGAT2(Maole_010035) |
| PEX10-35-R | caacgtggggttagtcgatcttcacgaactc | |
| PEX10-49-f | aatcatttaaccccacgttgccggtctt | PYL-Maole016461_PEX10 |
| PEX10-49-r | tctcggccatctgcggttagtactgcaaaaagtgc | |
| PEX10-49-F | ctaaccgcagatggccgagactgaagtg | pMV - MoDGA T2(Maole _015949) |
| PEX10-49-R | caacgtggggttaaatgattttcagttctctatcgg | |
| PEX10-88-f | ctggcagtaaccccacgttgccggtctt | PYL-Maole016461_PEX10 |
| PEX10-88-r | cgaagtccatctgcggttagtactgcaaaaagtgc | |
| PEX10-88-F | ctaaccgcagatggacttcgtgcccgac | pMV-MoGPAT(Maole_006088) |
| PEX10-88-R | caacgtggggttactgccagggctgagac | |
| PEX10-89-f | cacctcttaaccccacgttgccggtctt | PYL-Maole016461_PEX10 |
| PEX10-89-r | acagcaccatctgcggttagtactgcaaaaagtgc | |
| PEX10-89-F | ctaaccgcagatggtgctgtggcagtgc | pMV-MoGPAT(Maole_006089) |
| PEX10-89-R | caacgtggggttaagaggtggggggagag | |
| PEX10-90-f | tcgacgataaccccacgttgccggtctt | PYL-Maole016461_PEX10 |
| PEX10-90-r | gcagctgcatctgcggttagtactgcaaaaagtgc | |
| PEX10-90-F | ctaaccgcagatgcagctgcgaatcctg | pMV-MoGPAT(Maole_006090) |
| PEX10-90-R | caacgtggggttatcgtcgagagcaacatc | |
| TEFin-PF | AATTGCCCCAATTGACCC | |
| XPR2-PR | GGCATCTCACTTGCATATGTAT | |

PCR amplification of transformed fragments using 10up and 10dn on recombinant plasmids pYLEX-PEX10- MoDGAT2(Maole_010035)-URA, pYLEX-PEX10- MoGPAT(Maole_006088)-URA, pYLEX-PEX10-MoGPAT(Maole_006089)-URA, pYLEX-PEX10-MoGPAT(Maole_006090)-URA. All PCR amplifications were performed using KAPA HiFi high fidelity DNA polymerase in a 50 µl amplification system (2× KAPA Mix, 25 ul; 1.5 ul of each 10 µM primer; 1 µl of template; water supplemented to 50 µl). Each of the DNA sequences were obtained. Amplification conditions were: pre-denaturation at 95°C for 3 min; denaturation at 98°C for 20 s, annealing at 60-72°C for 15 s, extension at 72°C, extension times are based on 30 seconds per kb, with a number of cycles of 29-35; extension at 72°C for 10 minutes.

The PCR results are confirmed by agarose gel electrophoresis, the gels were cut and the transformed fragments are recovered and then LiAc transformation was used to transform the Yarrowia lipolytica Po1g-G3-CgKCS, which already expresses the elongase CgKCS, coated on screening plates in YNB-Δura medium and incubated at 28°C for 2-4 days to obtain a recombinant strain introducing the relevant esterase gene. Transformation of pYLEX-PEX10-MoDGAT2(Maole_010035)-URA" pYLEX-PEX10-MoDGAT2(Maole_015949)-URA, pYLEX-PEX10-MoGPAT(Maole_006088)-URA, pYLEX-PEX10-MoGPAT(Maole_006089)-URA, pYLEX-PEX10-MoGPAT(Maole_006090)-URA strains are abbreviated (from left to right) as YL-35, YL-49, YL-88, YL-89, YL-90, respectively. The recombinant strains were validated using a diagnostic PCR method, a single colony was picked from the screening plate, scribed on a YPD plate and grown for 24 h. Fresh bacteria cells were picked and the genomic DNA of the recombinant strains was extracted using a Rapid Fungi Genomic DNA Isolation Kit (Sango Biotech) and the positive clones were verified using the primers described in Table 2 using the genomic DNA as a template. PCR amplification was carried out using EasyTaq DNA polymerase and all amplification systems were 20 µl (2 × EasyTaq Mix, 10 µl; 0.6 µl each of 10 µM primers; 1 µl of template; water was added to make up to 20 µl), and the amplified fragments were determined by agarose gel electrophoresis. The amplification conditions were: pre-denaturation at 94°C for 3 min; denaturation at 94°C for 30 s, annealing at 55°C for 30 s, extension at 72°C, the extension time is calculated as 60 seconds per kb with a number of cycles of 36; extension for 10 minutes at 72°C.

**Table 2. Gene fragments and primers used for diagnostic PCR**

| Primer name | Primer sequences |
|---|---|
| 10up | gaagatcacgtggaagaagtac |
| 10dn | ccattcggaaatacagtcccaac |
| PEX10-df | acttgaagaggtatttgaaggtcac |
| PEX10-dr | tgataggcaacaagttctgc |
| 35-ndr | gaaagaagtggcggggttcaccag |
| 35-ndf | gaggacgactgcgccgac |
| 49-ndr | caggaaggtggcggtgaacatcac |
| 49-ndf | gaactctcagcccaccgcc |
| 88-ndr | cttctctcacctcagaagagccat |
| 88-ndf | cacgactctgtgaccgagcag |
| 89-ndr | ccagagtccagtccagcagc |
| 89-ndf | cgtgcccgacatttctgcc |
| 90-ndr | gcagttcagcaggaaagccg |
| 90-ndf | gctgttttcgactgcagcgag |
| INO2-ndr | cggaaagtcgtcttcttcggagg |
| INO2-ndf | cgaaacaaggccatgcggg |
| 1206-ndr | gtagtgacacaaacgaatttcctc |
| 1206-ndf | gccgagttgacatcttcctacgag |
| 35-ndr | gaaagaagtggcggggttcaccag |
| 35-ndf | gaggacgactgcgccgac |
| 49-ndr | caggaaggtggcggtgaacatcac |
| 49-ndf | gaactctcagcccaccgcc |
| TEFin-PF | aattgccccaattgaccc |
| XPR2-PR | ggcatctcacttgcatatgtat |

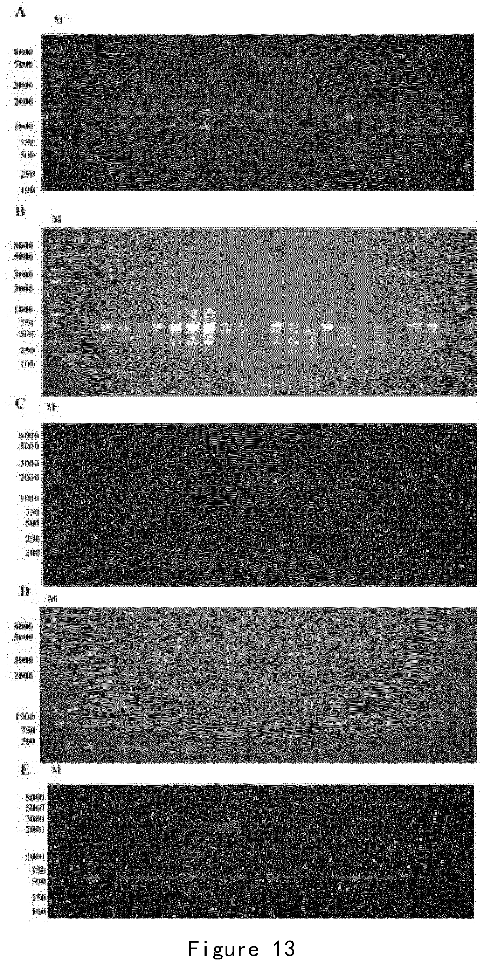

The recombinant strains were picked from the screening plates on YPD plates, YL-35, YL-49, YL-88, YL-89 and YL-90 were picked 48 each, while the control strains were inoculated on YPD plates and genomic DNA was extracted from the recombinant and control strains after approximately 24 hours of growth. The genomic DNA was used as a template for PCR amplification of the introduced genes to verify the integrated gene expression. Five positive clones were selected from these and named YL-35-F5 (co-expressing CgKCS and MoDGAT2 (Maole_010035)), YL-49-C6 (co-expressing CgKCS and MoDGAT2 (Maole_015949)), YL-88-B1 (co-expressing CgKCS and MoGPAT (Maole_ 006088), YL-90-B6 (co-expressing CgKCS and MoGPAT (Maole_006090)), as shown in Figure 13, wherein the CK strain, a Yarrowia lipolytica Po1g-G3-CgKCS expressing the elongase CgKCS, was subjected to subsequent shake flask fermentation, with the starting strain as a control.

### Example 4 Effects of esterases on oils and nervonic acid in malania oleifera

The strain and culture conditions were the same as in Example 1, 1.1.

The specific methods and steps for shake flask fermentation culture are the same as in part 1.2.5 of Example 1.

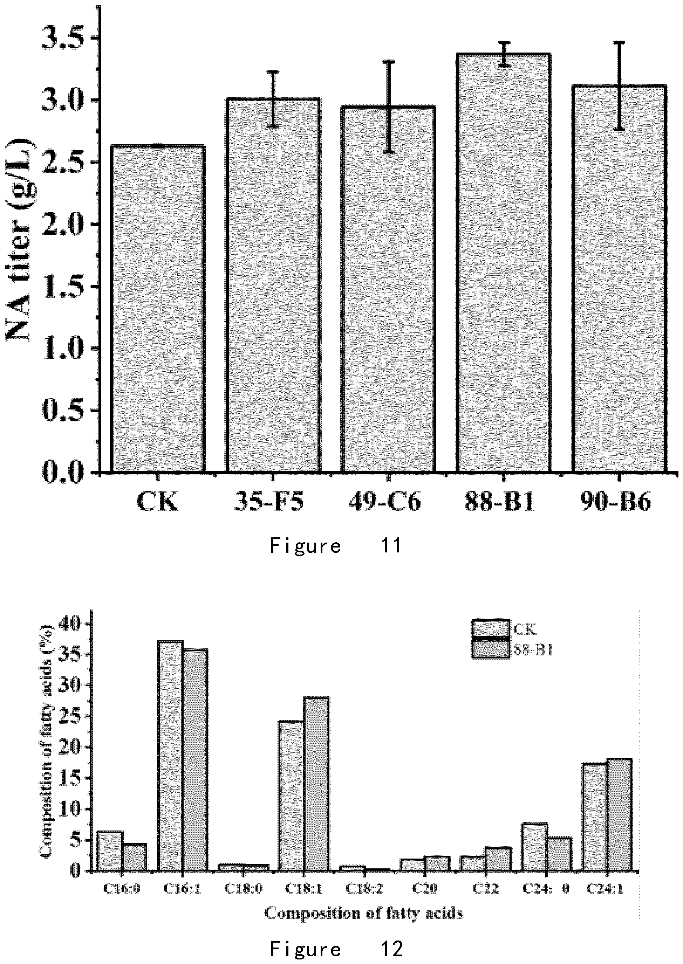

The oil extracted from YL-35-F5, YL-49-C6, YL-88-B1 and YL-90-B6 after shake flask fermentation, YL-35-F5, YL-49-C6, YL-88-B1 and YL-90-B6 all show significant improvement compared to the control Po1g-G3-CgKCS (CK), with an increase of 15.6%, 17.2%, 22.1% and 18.7% respectively, and the results are shown in Figure 10. Combined with the proportion of nervonic acid to total oil, it can be seen that YL-35-F5, YL-49-C6, YL-88-B1 and YL-90-B6 all show a significant increase in nervonic acid production compared to the control. Among them, YL-88-B1 has improved the most, reaching 3.36 g/L of nervonic acid, an increase of 28.16% compared to the control, the results are shown in Figure 11. Compared to the starting strain Po1g-G3-CgKCS, the proportion of oleic acid to total oil has increased from 24.2% to 28.0% and the proportion of nervonic acid to total oil has increased from 17.3% to 18.1% for YL-88-B1. The fermentation concentration of nervonic acid is increased by 28.1% and the results are shown in Figure 12.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. An engineered strain for the production of nervonic acid and/or oils, the engineered strain having a gene expression cassette integrated in the genome, the gene expression cassette expressing a protein encoded by a 3-ketoacyl-CoA synthase (KCS) gene and/or an esterase gene.

2. The engineered strain of claim 1, wherein the engineered strain comprises a yeast.

3. The engineered strain of claim 1, wherein the engineered strain is selected from the group consisting of: Yarrowia lipolytica, Saccharomyces cerevisiae, methylotrophic yeast, pichia pastoris, Hansenula polymorpha, Kluyveromyces, candida, Cryptococcus magnus, Issatchenkia orientalis, rhodotorula, and a combination thereof.

4. The engineered strain of claim 1, wherein the 3-ketoacyl-CoA synthase gene is derived from *Malania oleifera, Cardamine graeca, Acer truncatum, Lunaria annua, Ximenia caffra, Tropaeolum speciosum, Xanthoceras sorbifolia, Brassica, Capsella.*

5. The engineered strain of claim 1, wherein the engineered strain is *Yarrowia lipolytica,* preferably *Yarrowia lipolytica* Polg-G3-MaoleKCS or *Yarrowia lipolytica* YL-88-B1.

6. The engineered strain of claim 1, wherein the 3-ketoacyl-CoA synthase (KCS) gene or the esterase gene is a codon optimized 3-ketoacyl-CoA synthase (KCS) gene or a codon-optimized esterase gene.

7. The engineered strain of claim 1, wherein the sequence of the 3-ketoacyl-CoA synthase (KCS) gene is shown in any of SEQ ID NO. 1-11.

8. The engineered strain of claim 1, wherein the esterase gene is selected from the group consisting of: Diacylglycerol acyltransferase (DGAT2), Glycerol-3-phosphate acyltransferase (GPAT) enzyme gene, and a combination thereof.

9. The engineered strain of claim 1, wherein the sequence of the esterase gene is as shown in any one of SEQ ID NO. 48, 49, 50, 51, 52.

10. A method for producing nervonic acid and/or oil, comprising the steps of:
(i) culturing an engineered strain of claim 1, thereby obtaining a fermentation product containing nervonic acid and/or oil; and
(ii) isolating the nervonic acid and/or the oil from the fermentation product.

11. A method for constructing an engineered strain of claim 1, comprising the steps of:
(a) constructing a vector comprising a gene expression cassette, the expression cassette having the following elements: a selective marker gene, a resistance gene element, an rDNA homologous recombinant gene fragment, and a target gene, the target gene is a 3- ketoacyl -CoA synthase (KCS) gene and/or an esterase gene;
(b) transferring the vector containing the gene expression cassette obtained in step (a) into a recipient strain, thereby obtaining a strain in which the recipient genome is integrated with the gene expression cassette.

12. Use of an engineered strain of claim 1, wherein the engineered strain is used as a strain for fermentative production of nervonic acid and/or oil.
